(19) Europäisches Patentamt
European Patent Office
Office européen des brevets

(11) EP 2 560 695 B1

(12) **EUROPEAN PATENT SPECIFICATION**

(45) Date of publication and mention of the grant of the patent:
**21.02.2018 Bulletin 2018/08**

(21) Application number: **11714992.2**

(22) Date of filing: **19.04.2011**

(51) Int Cl.:
***A61L 29/08*** *(2006.01)* ***A61L 29/14*** *(2006.01)*

(86) International application number:
**PCT/EP2011/056246**

(87) International publication number:
**WO 2011/131677 (27.10.2011 Gazette 2011/43)**

(54) **PHOTOACTIVATED POLYMERIC MATERIAL AND ITS USE FOR THE PREPARATION OF MEDICAL DEVICES**

LICHTAKTIVIERTES POLYMERMATERIAL UND SEINE VERWENDUNG ZUR HERSTELLUNG VON MEDIZINISCHEN VORRICHTUNGEN

MATÉRIAU POLYMÈRE PHOTOACTIVÉ ET SON UTILISATION POUR LA PRÉPARATION DE DISPOSITIFS MÉDICAUX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.04.2010 PCT/EP2010/055107**

(43) Date of publication of application:
**27.02.2013 Bulletin 2013/09**

(73) Proprietor: **Invatec S.p.A.**
**25030 Roncadelle (BS) (IT)**

(72) Inventors:
- **D'ONOFRIO, Simone**
  **I-25030 Roncadelle, Brescia (IT)**
- **PELLEGRINI, Paolo**
  **I-25030 Roncadelle, Brescia (IT)**

(74) Representative: **Zimmermann & Partner Patentanwälte mbB**
**Postfach 330 920**
**80069 München (DE)**

(56) References cited:
**EP-A1- 0 633 031**
**WO-A1-2004/056909**
**WO-A1-2010/149719**
**WO-A2-2007/056338**
**US-A- 5 258 041**

- **SMOKAL V., KRUPKA O., WILCZEK M., KOSTRZEWA M., KOLENDO A.: "Study of the surface modification of polyethylene films via photochemical reaction with azidosulfonamides", DIGEST JOURNAL OF NANOMATERIALS AND BIOSTRUCTURES, vol. 3, no. 1, March 2008 (2008-03), pages 41-47, XP002614287,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 560 695 B1

## Description

The present invention relates to a medical device.

In particular, the present invention relates to a catheter having a surface endowed with improved properties.

More in particular, the present invention relates to a catheter tube having a surface endowed with improved properties.

Even more in particular, the present invention relates to a catheter tube of a catheter for endovascular interventions, said catheter tube having a surface endowed with improved properties.

According to an embodiment, the present invention relates to a catheter guide wire tube having an inner surface endowed with a low friction coefficient.

According to a different embodiment, the present invention relates to an inflation tube or an aspiration tube of a catheter for endovascular interventions, said inflation or aspiration tube having an inner surface endowed with improved properties.

Nowadays endovascular interventions are widely accepted procedures for treating different types of vascular diseases. For instance, angioplasty is a procedure for opening narrowed or even blocked blood vessels and restoring the normal blood flow. Angioplasty is successfully used also for treating coronary arteries, e.g. for treating coronary stenoses present therein.

For carrying out an endovascular procedure, a catheter (e.g. an angioplasty catheter) is inserted into a blood vessel of the patient (the blood vessel of an arm or of the groin, for instance) and then pushed to the intervention site, e.g. an occluded or partially occluded blood vessel (vein or artery). In order for the catheter to be correctly placed, typically a guide wire is firstly inserted into the blood vessel (optionally in combination with a guiding catheter) for allowing the correct positioning of the endovascular catheter, such as a balloon catheter, a stent delivery system, a clot extraction catheter, a multipurpose catheter. In case of an angioplasty procedure, the subsequent operations of inflating and deflating the balloon result in the plaque obstructing the vessel to be flattened against the vessel inner wall for re-establishing an acceptable blood flow.

There are continuous efforts in the art in order to improve the characteristics of the devices for endovascular procedures, in particular the size, flexibility, pushability and slidability properties.

Smokal et al., Digest Journal of Nanomaterials and Biostructures, Vol. 3, No. 1, (2008), p.41-47, relates to a study of the surface modification of polyethylene films via photochemical reaction with azidosulfonamides.

WO 2007/056338 A2 relates to ultra-thin photo-polymer coatings and uses thereof.

WO 2004/056909 A1 relates to a hydrophilic coating and a method for the preparation thereof.

EP 0633031 A1 relates to a composition for coating a surface of an implantable device, which composition inhibits formation of scar tissue at an in vivo site when the device is inserted at the site. The composition comprises a biocompatible polymer and an extracellular matrix molecule or fragment thereof.

Size is a crucial factor due to the very small dimensions of the vessels they have to be inserted in, especially when coronary arteries are treated. Small size, in terms of device diameter, is highly desirable in order to avoid damages to the vessel wall and for enhancing the patient vascular system compliance.

Flexibility must also be met, so that the device may suitably follow the vessel path, especially in case of particularly tortuous vessels.

Pushability refers to the possibility of the catheter to be easily pushed by the physician through the vascular system to the intervention site, while slidability relates to the capability of the catheter (and specifically of the catheter tube) to suitably slide over the guide wire.

As far as slidability is concerned, it is preferred that a negligible friction exists between the inner surface of the catheter (specifically the inner surface of the catheter tube) and the outer surface of the guide wire, so that the operation of inserting and extracting the guide wire into and from the catheter may be easily performed by the physician with no relevant efforts and reducing to a minimum the risk of damaging the vessel wall or of having the guide wire blocked inside the catheter, fact which would inevitably require the endovascular procedure to be interrupted.

In order to reduce the friction and to improve the slidability property of the endovascular catheter over the guide wire, it is known to coat the outer surface of the metallic guide wire with PTFE (polytetrafluoroethylene), a thermoplastic polymer having a very low friction coefficient.

Slidability can be improved also by operating on the catheter tube material and/or structure.

For instance, a multilayered catheter guide wire tube can be used, the outer layer of which provides for the requested mechanical resistance while the inner layer provides for the necessary slidability of the guide wire tube over the guide wire. An example of a multilayered guide wire tube structure is a tri-layered guide wire tube formed of PEBA-polyethylene resin-HDPE where the HDPE layer is the tube inner layer.

Alternatively, a mono-layered polyamide 12 (PA12) catheter guide wire tube can be used since polyamide 12 is endowed with suitable mechanical properties in terms of flexibility, chemical resistance, robustness, as well as with welding and workability properties. However, polyamide 12 has poor slidability properties.

Furthermore, at least two main procedures for the surface modification of polymers are known in the art.

According to a first procedure, the surface of a polymeric material can be modified by acting on its composition and/or microstructure.

According to a second procedure, a coating of a material endowed with the required characteristics is applied onto the polymeric material surface to be modified.

For instance, US 5,714,360 discloses the covalent linkage of a target molecule, selected among synthetic polymers, especially polyvinylpirrolidone, carbohydrates, proteins, lipids, nucleic acids, drugs, dyes and fluorescent compounds, capable of conferring a particular property to a substrate through a photoactivating linking agent. US 6,551, 267 discloses a plastic material article coated with a crosslinked hydrogel permanently bound to the inner wall of the tube. US 6, 120, 904 discloses the formation of a polyurethane/polyurea hydrogel coating onto a plasma treated surface. US 5,415,619 describes the modification of a polyester surface with the reaction of sulphuric acid which originates negative charges; the further treatment with perchloric acid leads to the insertion of hydrophilic functional groups like hydroxyl groups. Polyamide surfaces may also be modified according to the method disclosed in Polymer (2006, vol.47, 14, 4916-4924) with the use of a strong base like potassium terbutylate; the potassium salt thus formed may bind other useful molecules.

The method of applying a coating may be performed by dipping or spraying as taught, for instance, in US 5,061,738 wherein a mixture of a silicone resin and heparin is applied to a tube surface.

Alternatively, extrusion and co-extrusion techniques may be used, wherein a lubricating hydrophilic polymer immiscible with the tube material and the tube material are co-extruded, as disclosed for instance in US 6,506,333.

The article "To adjust wetting properties of organic surface by in situ photoreaction of aromatic azide" published by Feng Shi et al. on Langmuir 2007, 23(3), 1253-1257, discloses an alkyl or substituted alkyl chain, such as halogen substituted chain, that may impart hydrophobic property to a modified polymeric surface.

The article "Adsorption of proteins onto poly(ether urethane) with a phosphorylcholine moiety and influence of preadsorbed phospholipid" published by A.P. van der Heiden et al., Journal of Biomedical Materials Research (1998), 40(2), 195-203, discloses a PEU film that is photochemically modified with a PC-containing aryl azide.

However, none of the prior art methods mentioned above is suitable for giving the desired hydrophilic or hydrophobic properties to the surface of a catheter (in particular, the inner surface of a catheter tube) without negatively affecting the other performances thereof.

In fact, the methods which involve the application of a coating onto a device surface, and in particular onto the inner surface of a device in form of a tube, have the drawback of increasing the thickness of the tube wall, thereby causing a decrease of the inner lumen of the tube suitable for inserting and extracting the guide wire, operations which may become more difficult. Clearly, the manufacture of a larger catheter (i.e. with increased outer diameter) for overcoming the increased wall thickness is not feasible due to the limited vessel dimensions.

Furthermore, the applied coating being not very stable, can be removed, damaged or degraded, such as delaminated or peeled, while the tube is being used.

On the other hand, plasma treatments, which modify the surface of the tube material, may cause damages to the surface itself and do not provide reproducible results.

The use of chemical reactions for the introduction of functional groups into the polymeric material may lead to structural alterations of the surface and sometimes an insufficient functionalisation is obtained.

Finally, the compounding techniques are complex and difficult to set up in order to obtain reproducible results. Moreover, said techniques are quite expensive, too.

Accordingly, the Applicant has perceived the need of providing a reliable and reproducible method which is suitable for modifying the surface of a medical device, in particular the inner surface thereof.

In particular, the Applicant has perceived the need of providing a reliable and reproducible method which is suitable for decreasing the friction coefficient of the surface of a medical device, particularly of the inner surface of a catheter tube.

## OBJECT OF THE INVENTION

Subject matter of the present invention is a medical device as defined in claim 1. The dependent claims relate to particular embodiments thereof.

Further subject matter of the present invention is a method for modifying the surface of the medical device as defined in claim 7. The dependent claims relate to particular embodiments thereof.

Further subject matter of the present invention is a compound as defined in claim 11, and the use of the compound for modifying the properties of a polymeric surface, as defined in claims 12 to 14.

It is an object of the present invention a medical device, particularly a catheter, an inner surface of which is prepared via the photoactivation with substituted aromatic azides.

In particular, said photoactivation may impart either hydrophobic or hydrophilic properties to the surface of a

catheter.

It is a further object of the invention to modify the inner surface of a catheter tube of an endovascular catheter through the photoactivation of said inner surface with substituted aromatic azides.

According to a still further embodiment, the present invention discloses the use of substituted aromatic azides for the photoactivation of polymeric surfaces.

BRIEF DESCRIPTION OF THE FIGURES

Fig. 1 shows the mechanism of insertion of the para-substituted arylazide compounds of the invention into a C-H containing surface.

Fig. 2 shows the TLC obtained for the preparation of a compound according to EXAMPLE 4.

Fig. 3-8 show the spectra obtained with XPS analysis of samples treated according to the present invention.

Fig. 9 shows the reaction mechanism for light excitation of arylazides of the invention.

Fig. 10 shows the TLC obtained for the preparation of the compounds according to EXAMPLE 14.

DETAILED DISCLOSURE OF THE INVENTION

According to the first embodiment of the present invention a medical device, particularly a catheter, comprises a hollow body made of a polymeric material and has an outer and an inner surface, wherein said inner surface has been modified by the insertion of hydrophilic or hydrophobic moieties.

In particular, said catheter is a catheter guide wire tube, an inflation tube of a balloon catheter or an aspiration tube.

In the present description and claims the term "guide wire tube" includes any catheter component which defines and possesses a lumen suitable for receiving a guide wire there into. In detail, in case a coaxial structure is considered, the guide wire tube extends for the whole catheter length and it is coaxially arranged with respect to the catheter shaft, the guide wire tube being internal to the catheter shaft. Alternatively, in case a bilumen structure is considered, the guide wire tube includes the shaft catheter portion forming the guide wire lumen and the actual guide wire tube starting at the balloon proximal end.

In the present description and claims the term "inflation tube" includes any catheter component which defines and possesses a lumen suitable for receiving an inflation fluid which allows inflation and deflation of a catheter balloon.

With "aspiration tube" it is intended any catheter component which defines and possesses a lumen suitable for the aspiration of clots, thrombi, ....

In the present description and claims the term "inner surface" of a catheter tube, such as a catheter guide wire tube, a balloon catheter inflation tube or an aspiration tube of a clot extraction catheter, refers to the surface of a lumen which is suitable for receiving a guide wire, an inflation fluid or an aspirated material (clot, thrombi, ..), respectively.

According to another object of the present invention a medical device as above disclosed has, in addition or in alternative to the inner surface ,the outer surface which is modified by the insertion of hydrophilic or hydrophobic moieties.

In detail, in case the catheter tube is a guide wire tube as above defined, the friction under consideration is the one between the guide wire outer surface and the guide wire tube inner surface (inside of which the guide wire is caused to slide). Since, as mentioned above, the guide wire is usually provided with a hydrophobic PTFE coating, according to the present invention the inner surface of the guide wire tube is photoactivated with substituted aromatic azides suitable for imparting hydrophobic properties to said inner surface. In this way, in fact, the friction between the two contacting hydrophobic materials can be kept advantageously low.

Alternatively, in case the catheter tube is an inflation tube as above disclosed, particularly a balloon catheter for angioplasty, the Applicant has noted that the inflation tube inner surface modified according to the present invention advantageously improves the distribution and the sliding of the inflation fluid along the inflation tube. This aspect favourably affects the balloon inflation and deflation times, which can be advantageously reduced. A decrease of the balloon inflation time, and especially of the balloon deflation time, is particularly advantageous for applications where long balloons (e.g. up to 300mm) or valvuloplasty balloons, which have relevant radial dimensions in comparison with the longitudinal ones, are used.

Moreover, in case a coaxial structure as better defined in the following description is considered, both the guide wire tube and the inflation tube inner surfaces may be modified by the insertion of hydrophilic moieties. Thus, the inflation fluid, which moves in the space between the inflation tube (catheter shaft) and the guide wire tube, is advantageously made sliding with low friction forces.

Furthermore, hydrophilic properties may also be imparted according to the present invention to the inner surface

of an aspiration tube of a medical device, such as a clot extraction catheter. In fact, the Applicant has noted that the aspiration tube inner surface thus modified advantageously contributes in increasing the aspiration efficiency of the aspiration catheter, especially in case of very dense material (clots, thrombi, ...) to be aspirated from a blood vessel.

According to the present invention, the inner surface of a guide wire tube as above disclosed may be modified in order to impart hydrophilic properties. In this way, in fact, due to the cushioning effect produced by the presence of a thin liquid layer, the friction between the latter and the hydrophilic inner surface of the guide wire tube is advantageously and surprisingly low. In fact, before the endovascular procedure is started, the guide wire is wetted with a liquid solution, typically an aqueous solution of heparin, in order to avoid that blood coagulation occurs on the guide wire outer surface. The modified inner surface of the guide wire tube according to the invention is capable of attracting the wetting solution and generating the thin liquid layer above mentioned.

In the present description and claims the terms "hydrophilicity" and "hydrophobocity" refer, respectively, to high and low affinity with water of a given molecule or compound. These properties are generally evaluated by measuring the contact angle ($\theta$) as better explained in the Experimental Section of the present description. Typically, the range limits are $\theta$=0°, indicating complete wettability and thus maximum hydrophilicity, and $\theta$=180°, indicating absence of wettability and thus maximum hydrophobicity. For example, PA12 has a contact angle $\theta$ of about 85°; therefore, a surface modification which increases this angular value makes the PA12 material more hydrophobic while a surface modification which decreases this angular value makes the PA12 material more hydrophilic. Examples of hydrophilic groups are -OH, -COOH, $-SO_3$, $-PO_4$, $-NH_2$, $-NH_4^+$, PEG (poly(ethylene glycol)), PEO (polyethylene oxide). Examples of hydrophobic groups are aliphatic carbon chain, such as, for instance, hydrocarbon chains, polyethylene, polypropylene and polyolefin in general, or an aromatic group, such as, for instance, xylene, polystyrene, acrylonitrile butadiene styrene, or fluoropolymers of formula $(CF_2)_nCF_3$ wherein n= from 1 to 70, such as, for instance, polytetrafluoroethylene (PTFE).

As the present invention finds particular application for the preparation of medical devices, biopolymers are particularly suitable as the polymeric material for manufacturing the catheter tube.

Within the following disclosure, a biocompatible polymeric material or a biopolymer is intended to encompass those materials which may be suitably placed in contact with a body surface or tissue and especially with the blood, without triggering the formation of blood clotting or thrombi. Hydrophilic surfaces have in fact been demonstrated to slow the blood macromolecules and corpuscles absorption.

Preferably, the polymeric material which is suitable for the present invention comprises C-H or C-X functional groups, where X is an heteroatom. Preferably, X is selected from the group comprising nitrogen, oxygen, sulphur, phosphorous, boron, chloride, bromine and iodine.

Particularly preferred biocompatible polymers which are suitable for the present invention include, for instance, polyamides, polyester-polyamide copolymers, the polyamide-based copolymers of general formula

$$H\text{-}(O\text{-}PF\text{-}OOC\text{-}PA\text{-}COO\text{-}PF\text{-}OOC\text{-}PA\text{-}CO)_n\text{-}OH$$

wherein PA is a polyamide segment and PF is a diol segment comprising OH-terminating dimer diol polyesters and n is between 5 and 20 as disclosed in WO 2005/037337 or the polymeric material may be an elastomer obtained by the polymerization of a polyamide forming block compound selected in the group comprising an aminocarboxylic acid of formula (1) below and a lactam of formula (2) below

$$H_2N\text{-}R1\text{-}COOH \qquad (1)$$

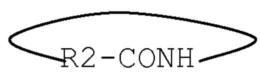 (2)

with a polyetherdiaminic triblock of formula (3) below:

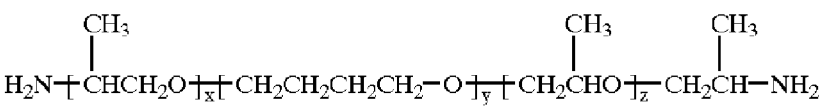 (3)

and a dicarboxylic acid of formula (4) below:

$$HOOC\text{-}(R_3)_m\text{-}COOH \qquad (4)$$

wherein R1, R2 and R3 are each binding groups comprising a hydrocarbon chain therein, which may be interrupted by one or more amide groups and wherein R1 and R2 comprise independently an alkylene group having 2 to 20 carbon atoms and amide bonds and R3 comprises an alkylene group having 1 to 20 carbon atoms and wherein x may change from 1 to 20, preferably from 1 to 18, more preferably from 1 to 16, wherein y may change from 4 to 50, preferably from 5 to 45, more preferably from 8 to 30 and z may change from 1 to 20, preferably from 1 to 18, more preferably from 1 to 12 and wherein m is 0 or 1 as disclosed in WO 2007/132485, whose content, with respect to the compounds and preparation methods, is herewith incorporated by reference.

A particularly preferred polymer of the present invention is polyamide and, more in particular, polyamide PA12 and PEBAX®.

According to the present invention, the modification of the polymer surface is obtained by the insertion of moieties capable of modifying the wetting properties of the material surface. As shown in Figure 1, a substituted aromatic azide bearing a R group under UV light produces the extremely reactive intermediate phenylnitrene. Then, the nitrene radical takes an hydrogen from a substrate having C-H bonds thus giving two radical moieties, which then combine together. There results that the azide molecule is inserted into the substrate.

According to an embodiment of the present invention, the polymeric inner surface of a catheter tube is rendered hydrophilic, or at least more hydrophilic than before, by covalently bounding to said inner surface groups of formula

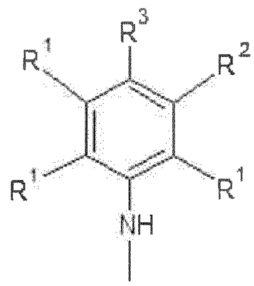

wherein $R^1$ and $R^2$ independently from each other are H or F, and $R^3$ is a Z group; or
wherein each $R^1$ independently is H or F, $R^2$ is a Z group, and $R^3$ is $-NO_2$;
wherein the Z group is selected from $-C(O)NH-R_a$, $-S(O)_2NH-R_a$ and $-P(O)_2NH-R_a$ wherein $R_a$ is $C_1$-$C_4$ linear or branched saturated alkyl chain optionally substituted with one or more polar functional groups selected from -OH, -COOH, $-SO_3$ $-PO_4$, $-NH_2$, $-NH_4+$ or with a $-(CF_2)_m-CF_3$ perfluoroalkyl group wherein m is 1 to 70; or wherein $R_a$ is $-(CHRCH_2O)_n-X$ wherein n is 1 to 70, R is H or $-CH_3$ and X is selected from H, saturated branched or linear $C_1$-$C_4$ alkyl chain or a $-(CH_2)_p-O-(CH_2)_q-W$ group wherein W is H, $-CH_3$ or $-NH_2$ and wherein p and q are independently 1 to 30; or wherein $R_a$ is a $C_1$-$C_{70}$ linear or branched saturated alkyl chain or an aromatic group.

As per $R_a$ being a linear or branched saturated alkyl chain, it can be selected from the group comprising polyethylene, polypropylene and polyolefins, while when $R_a$ is an aromatic group, it can be selected among the group comprising xylene, polystyrene and acrylonitrile butadiene styrene.
A preferred perfluoroalkyl $R_a$ group is $-(CF_2)_nCF_3$, wherein n is 1 to 70 or polytetrafluoroethylene.

As per a preferred embodiment of the invention, N-(tris(hydroxymethyl)-4-azidobenzenesulphonylamide, N(-2-hydroxyethyl)-4-azidobenzamide, N-(2-hydroxyethyl)-4-azidobenzenesulphonylamide, N-(Jeffamine® M-600)-4-azidobenzamide, 4-azido-2,3,5,6-tetrafluoro-N-(3-hydroxypropyl)benzamide, 4-azido-2,3,5,6-tetrafluoro-N-(4,4,5,5,6,6,7,7,8, 8,9,9,10,10,11,11,11-heptadecafluoroundecyl) benzamide, 2-nitro-5-azidobenzoylglycine, N-(4,4,5,5,6,6,7,7,8,8,9,9, 10,10,11,11,11-heptadecafluoroundecyl) 5-azido-2-nitrobenzoate, N-(tris(hydroxymethyl)-5-azido-2-nitrobenzoate and N-(dodecaethylene glycol monomethyl ether)-5-azido-2-nitrobenzoate have been used for the surface treatment of the catheter tube polymeric material. The preparation of said compound is detailed in the following Experimental Section.

Nevertheless, by changing the composition of the para-substituting R group, in place of the hydrophilic groups mentioned above, suitable hydrophobic moieties can be introduced on the inner surface of the catheter tube.

In fact, as already mentioned above, while a hydrophilic treatment is normally preferred, the sliding properties of the inner surface of a catheter tube can also be improved by carrying out a hydrophobic treatment. The latter, in fact, allows to minimise the interaction between the catheter tube inner surface and the fluid layer surrounding the guide wire, so that the catheter tube suitably and advantageously slides over said layer.

The Applicant has found that in spite of the fact that the surface to be treated is not exposed to light - being an inner surface of a catheter tube - the photoactivation can be successfully carried out. To this aim, it is necessary to select the wavelength of the UV light source as well as the polymeric material to be treated so that no absorbance of the radiation may occur. It has been found that the photoactivating wavelength of the above azide compounds does not

interfere with the absorbance wavelength of the materials normally used for catheter tubes.

According to an embodiment of the present invention, an alcoholic solution of methanol or ethanol or acetone or an acetonitrile or a chloroform solution of a para-substituted arylazide compound is prepared and the inner surface to be treated is contacted with said solution. For instance, the solution may be made flowing inside the catheter tube, i.e. through the catheter tube lumen. Then, the treated surface is activated by light of a suitable wavelength capable of photoactivating the para-substituted arylazide and irradiation is performed for a suitable period of time. Preferably, the light wavelength for a given para-substituted arylazide is selected as the wavelength which substantially corresponds to the maximum absorption for that specific para-substituted arylazide. Typically, the light wavelength is comprised from about 200 nm to about 600 nm. Preferably, the light wavelength is comprised from about 250 nm to about 350 nm and even more preferably is comprised from about 230 nm to about 300 nm. The photoactivation reaction is carried out in a dark environment, at room temperature, i.e. about 25°C, and the reaction time is typically comprised from 0.5-1 hour.

As above disclosed, the outer surface of a medical device can be modified in order to impart hydrophilic or hydrophobic properties.

The Applicant has also found that the photoactivation reaction can also occur by causing the solution of azide compound to flow continuously within the catheter tube. This aspect is particularly advantageous since a continuous process avoids all the drawbacks that are typical of a batch process, in particular in terms of time and costs saving, as well as in terms of uniformity and homogeneity of the treated surface.

Before the photoreaction treatment, the catheter tube inner surface is preferably washed in order to remove any impurities from the surface. Typically, after the photoreaction treatment has occurred, the catheter tube inner surface is washed again until all the unbound molecules have been removed. Monitoring of the washing solution is generally carried out by UV.

As said above, according to the present invention, it is the inner surface of a catheter tube - e.g. a guide wire catheter tube, an inflation tube of a balloon catheter or an aspiration tube of a clot extraction catheter - which is modified. Thus, it is necessary that the irradiating UV light passes through the polymeric material of the catheter tube and reaches the catheter tube inner surface where the activation process occurs. Therefore, the polymeric material of the catheter tube must be transparent to the specifically used UV irradiating light.

Preferably, the light wavelength is comprised within the visible spectra and is selected according to the activating molecule used. According to an embodiment of the present invention, the wavelength of maximum absorbance of the substituted arylazide compound is used. For instance, a 254 nm wavelength can be advantageously used since the polymeric materials typically used in the manufacture of catheter tubes are transparent to this light wavelength.

The extent of the modification of the inner surface of the catheter tube treated according to the present invention as well as the modification of the wetting properties of said inner surface may be evaluated by measuring the contact angle with the CAM system of KSV Instruments or other similar systems as described in the following Example 5.

The modification of the composition of the inner surface material of the catheter tube treated according to the method of the present invention may be analysed by XPS (Photoelectron Spectroscopy) or ESCA (Electron Spectroscopy for Chemical Analysis).

Example 1

Preparation of N-(tris(hydroxymethyl)-4-azidobenzenesulphonylamide

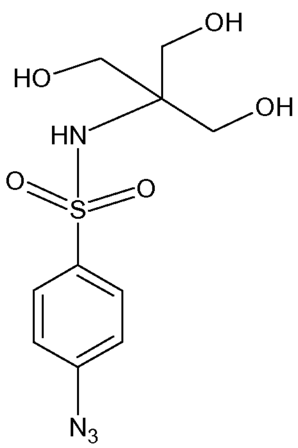

The first step for the preparation of the title compound followed the disclosure of G.A. Wiese e J. W. Jones "Preparation of Tris (hydroxymethyl) sulfanilamidomethane", Journal of the American Pharmaceutical Association,S.E., 380-383 (1948).

In a two-necked flask, 2 moles of 2-amino-2(hydroxymethyl)-1,3-propanediol (TRIS) were solubilised in a minimum amount of water. Then, 1 mole of acetamidobenzenesulphonyl chloride was added. The reaction was left at 70°C for about half an hour checking the reaction by TLC with t-butanole-ethyl acetate 2:8 as eluting agent. The substituted amide was treated with a 4-6 N HCl solution in a 1/1.4 molar ratio at 70-80°C under reflux for about 5 hours. The reaction was checked by TLC with t-butanol-ethyl acetate 2:8 as eluting agent until the starting compound disappeared.

The solvent was then evaporated until a yellowish gel was obtained. The latter was treated with the minimum amount of ethanol in order to solubilise, except for the TRIS chloridrate. Then, water-heating was continued until complete solubilisation, followed by quenching and filtration. The alcoholic solution was treated with gaseous ammonia until a white precipitate of ammonia chloride was obtained. Then a filtration step was carried out and the filtered mixture was left overnight in a fridge.

A white precipitate of N-(tris(hydroxymethyl))-4-aminobenzenesulphanylamide was obtained, which was filtered and re-crystallized with an ethanol-water solution 9:1.
Melting point: 158°C (literature: 159-161°C)

The title compound was then prepared by solubilising the N-(tris(hydroxymethyl))-4-aminobenzenesulphanylamide in an aqueous solution of chloride acid (rate 5:1) and was stirred for 15 min in an ice bath at 0°C. Then, a solution obtained dissolving sodium nitrite in the minimum amount of water (nitrite:aniline rate 1:1) was added dropwise bringing the temperature to -5/-10°C with an ice bath. Then, the reaction was left for 30 min under stirring. The $NaN_3$ was solubilised (molar ratio sodium azide:aniline 1:3) into the minimum water amount and then quenched. It was then added dropwise to the solution and was left reacting for 1 hour at room temperature under stirring. A white precipitate formed, which was filtered and re-crystallized with aqueous methanol (1:1 solution).
Melting point: 125°C
**IR** *(NaCl)* 3280, 2110, 2130, 1590,1288,1054;
**H-NMR** *(DMSO)* (300 MHz):3.43 (s, 6H), 4.2 (br, 3H), 6.92 (s, 1H), 7.25 (2H, d), 7.87 (2H, d);
**C-NMR** *(DMSO)* :60.8 (CH2), 64,5 -119.2 (CH), 128.2 (CH), 140.7, 142.8;
**UV***(MeOH)*: 208 (4.06), 263 (4.07),290 (sh, 3.40)

EXAMPLE 2

Preparation of N(-2-hydroxyethyl)-4-azidobenzamide

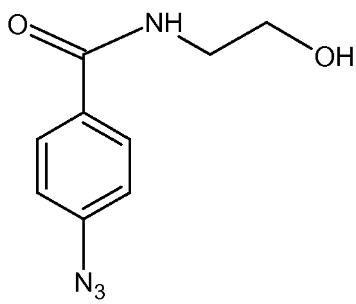

The synthesis followed the description of the process disclosed by H.Brintzinger and H. Koddebusch, "Amid- und Ester-amid-Bildung zwischen carbonsäurechloriden und Mono-, Di- und Triäthanolamin", Chemische Berichte 82, 201 (1949).

p-nitrobenzoylchloride was dissolved into the minimum quantity of chloroform and equivalent moles of ethanolamine in chloroform (about the same volume). The two solutions were added dropwise to a solution of 2 ml of chloroform within a flask under stirring conditions and under room temperature. The temperature was kept below 40°C with an ice bath. A white crystalline solid of 4-nitro-N-(2-hydroxyethyl)benzamide formed (yield 90%) which was filtered under pressure and re-crystallized (100% ethylacetate).
Melting point: 123°C (literature: 123-125°C)
Then, the 4-nitro-N-(2-hydroxyethyl)benzamide was solubilised in ethanol and Pd/C was added as the catalyst (Pd/C: 4-nitro-N-(2-hydroxyethyl)benzamide 1:5 weight). In a hydrogenation Erlenmeyer flask the catalyst was placed on the bottom flask, then the 4-nitro-N-(2-hydroxyethyl)benzamide and 25 ml of ethanol were added. The mixture was stirred

and connected to the hydrogenator. Hydrogen was fluxed into the alcoholic solution under stirring conditions and under atmospheric pressure. Hydrogenation was checked following the hydrogen consumption. At the end of the hydrogenation, filtration was made on filter paper and solvent removed with rotavapor. An oily residue was obtained which crystallized under cooling. A white solid was obtained which was crystallized in 100% ethyl acetate. A white crystalline solid of 4-amino-N-(2-hydroxyethyl)benzamide was obtained.
Melting point: 119°C (literature: 119-120°C)

The title compound was then obtained by solubilising the 4-amino-N-(2-hydroxyethyl)- benzamide into an aqueous solution of chloridic acid (rate 5:1) and was stirred for 15 min in an ice bath at 0°C. Then, a solution obtained dissolving sodium nitrite in the minimum amount of water (nitrite:aniline rate 1:1) was added dropwise bringing the temperature to -5/-10°C with an ice bath. Then, the reaction was left for 30 min under stirring. The $NaN_3$ was solubilised (molar ratio sodium azide:aniline 1:3) into the minimum water amount and then quenched. It was then added dropwise to the solution and was left reacting for 1 hour at room temperature under stirring. A white precipitate formed, which was filtered and re-crystallized with aqueous methanol (1:1 solution). Melting point: 97-98°C.
**IR** (NaCl):3305,2132,2110,1634,1284,1058;
**H-NMR** *(DMSO)* (300 MHz):3.30 (q, 2H), 3.50 (q, 2H), 7.2 (d, 2H), 7.9 (d, 2H), 8.45 (t);
**C-NMR** *(dmso)*:42.2 (CH2), 59.7 (CH2), 118.8 (CH), 129.1 (CH), 131.1, 142.1, 165.3;
**UV** *(MeOH) :211* (4.23) 268 (4.27) 290 (sh, 378)

EXAMPLE 3

Preparation of N-(2-hydroxyethyl)-4-azidobenzenesulphonylamide

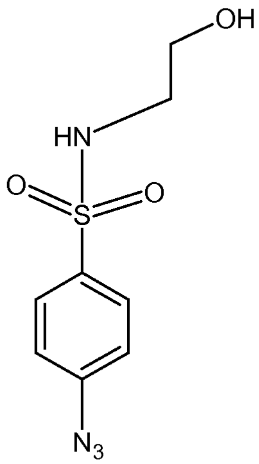

The synthesis followed the description of the process as disclosed by M.L. Crossley, E.H. Northey, M.E. Hultquist, "Sulfanilamide Derivatives. VI. Substituted N-Aliphatic Sulfaniamides" J. Am. Chem. Soc., 62 (3), pp 532-534 (1940) with some modifications.

p-nitrobenzenesulphonyl chloride was solubilised into the minimum amount of chloroform and equivalent moles of ethanolamine in chloroform (about the same volume).
The two solutions were added dropwise to a solution of 2 ml of chloroform within a flask under stirring conditions and under room temperature. The temperature was kept below 40°C with an ice bath. A red crystalline solid of N-(2-hydroxyethyl)-4-nitrobenzenesulphonamide formed (yield 68%) which was filtered under pressure and re-crystallized (100% ethylacetate).
Melting point: 127°C (literature: 126-127°C). N-(2-hydroxyethyl)-4-nitrobenzenesulphonamide was dissolved in ethanol into a Erlenmeyer flask and Pd/C was added as the catalyst (N-(2-hydroxyethyl)-4-nitrobenzenesulphonamide:Pd/C 5:1 weight ratio).
Then, in the Erlenmeyer flask the catalyst was placed on the bottom, then the N-(2-hydroxyethyl)-4-nitrobenzenesulphonamide and 25 ml of ethanol were added. The mixture was stirred and connected to the hydrogenator. Hydrogen was fluxed into the alcoholic solution under stirring conditions and under atmospheric pressure. Hydrogenation was checked following the hydrogen consumption. At the end of the hydrogenation, filtration was made on filter paper and solvent removed with rotavapor. An oily residue was obtained which crystallized under cooling. A brownish-yellowish solid was obtained which was re-crystallized in 100% ethyl acetate. A brownish-yellowish crystalline solid of N-(2-hydroxyethyl)-4-aminobenzenesulphonamide was obtained.
Melting point: 95°C (literature: 95-97°C)
The title compound was prepared solubilising the N-(2-hydroxyethyl)-4-aminobenzenesulphonamide into an aqueous solution of chloridic acid (rate 5:1) and was stirred for 15 min in an ice bath at 0°C. Then, a solution obtained dissolving sodium nitrite in the minimum amount of water (nitrite:aniline rate 1:1) was added dropwise bringing the temperature to -5/-10°C with an ice bath. Then, the reaction was left for 30 min under stirring. The $NaN_3$ was solubilised (molar ratio sodium azide:aniline 1:3) into the minimum water amount and then quenched. It was then added dropwise to the solution and was left reacting for 1 hour at room temperature under stirring. A white precipitate formed, which was filtered and re-crystallized with aqueous methanol (1:1 solution).
Melting point: 57-58°C.
**IR** (NaCl):3445,2134,2115,1588,1301,1160;
**H-NMR** *(dmso)* :2.77 (q, 2H), 3.36 (t, 2H), 4.8 (br, 1H), 7.32 (8d, 2H), 7.64 (t, 1H), 7.47 (d, 2H); **C-NMR** *(dmso):45.0* (CH2), 59.8 (CH2), 119.7 (CH2) 128,5 (CH2) 136.8, 143.5;
**UV** *(MeOH)* :210 (4.30), 263 (4.40) 290 (sh, 2.45)

EXAMPLE 4

Preparation of N-(Jeffamine® M-600)4-azidobenzamide

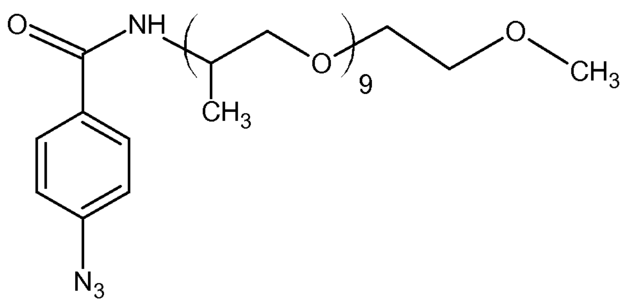

p-nitrobenzoylchloride was dissolved into the minimum quantity of chloroform and equivalent moles of Jeffamine® M600 (Jefferson Chemical Co.) of formula

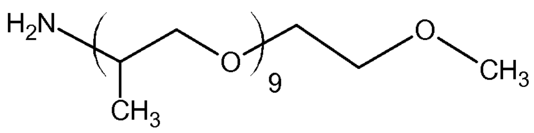

into chloroform. The two solutions were added dropwise to a solution of 2 ml of chloroform within a flask under stirring conditions and under room temperature. The reaction was left for 24 h checking the reaction by TLC in 100% ethylacetate until no p-nitrobenzoilchloride is detected in the reaction mixture. The solvent is evaporated with rotavapor and a yellowish syrup-like oil is obtained. By TLC (ethyl acetate/cycloesane 8/2) four reaction products are detected as shown in Fig.2, wherein line 1 corresponds to p-nitrobenzoilchloride and line 2 corresponds to the reaction products. The mixture is then separed by chromatography.
Chromatography column:

Diameter: 5 cm
Fixed phase: neutral alumina- 12 cm high
Mobile phase: cycloesane

The oil is solubilised into the minimum quantity of cycloesane and is deposited in the column.
The first eluted compound (Rf TLC: 0.87) is the white crystalline solid of ethyl-4-nitrobenzoate; the other three eluted products all are N-(Jeffamine® M-600)-4-nitrobenzamide.
**IR** (NaCl):3325, 2975, 1653,1602,1527,1346,1107;
**H-NMR** *($CDCl_3$)*:1.1 (m, ca. 21 H),3.3 (s, 3H),3.3-3.7 (m, ca. 22 H),8.0 (m, 2H),8.23 (d, 2H);

**C-NMR** *($CDCl_3$)*: ca. 17 (6 CH3),59.0 (CH3),71.5-73.4 (5 CH2),74.7-75.3 (5 CH),123.3,123.4 (CH), 18.4(3CH), 140.4 (2C), 149.3, 164.5, 164.8. N-(Jeffamine® M-600)-4-nitrobenzamide was solubilised in ethanol and Pd/C was added as the catalyst (N-(Jeffamine® M-600)-4-nitrobenzamide:Pd/C 5:1 weight ratio).

Then, in the Erlenmeyer flask the catalyst was placed on the bottom, then the N-(Jeffamine® M-600)-4-nitro benzamide and 25 ml of ethanol. The mixture was stirred and connected to the hydrogenator. Hydrogen was fluxed into the alcoholic solution under stirring conditions and under atmospheric pressure. Hydrogen was checked following the hydrogen consumption. At the end of the hydrogenation, filtration was made on filter paper and solvent removed with rotavapor. A yellowish-browinigh oil is obtained.

**IR** *(NaC)l:* 3355, 2972,1634,1608,1506,1105;

**H-NMR** (*$CDCl_3$)*:1.15-1.25 (ca. 30 H, m), 3.45 (s, 3H),3.3-3.8 (ca. 36 H, m),6.82 (m, 2H), 7.70 (m, 2H)

Then, N-(Jeffamine® M-600)-4-aminobenzamide was solubilised into an aqueous solution of chloridic acid (rate 5:1) and was stirred for 15 min in an ice bath at 0°C. Then, a solution obtained dissolving sodium nitrite in the minimum amount of water (nitrite:aniline rate 1:1) was added dropwise bringing the temperature to -5/-10°C with an ice bath. Then, the reaction was left for 30 min under stirring. The $NaN_3$ was solubilised (molar ratio sodium azide:aniline 1:3) into the minimum water amount and then quenched. It was then added dropwise to the solution and was left reacting for 1 hour at room temperature under stirring. Two phases formed by adding chloroform: a yellowish oil was obtained.

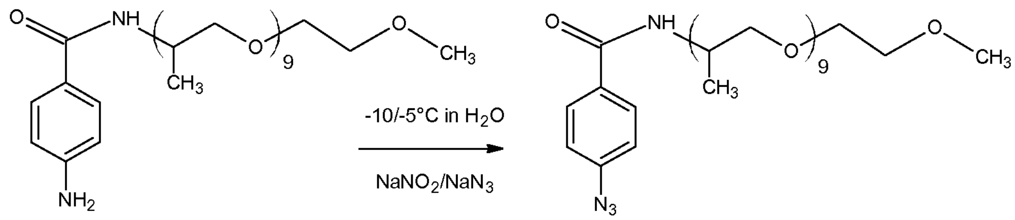


**IR** (NaCl):3350, 2972,2123,1654,1688,1604,1499,1275,1109;

**H-NMR** (*$CDCl_3$*): 1.15 (m, 21H),3.4 (s, 3H),3.4-3.7 (m, 23 H),7,4 (d, 2H) 7.8-7.9 (m, 2H);

**C-NMR** *($CDCl_3$)*: 17 (6 CH3), 58.9 (CH3), 72 (5 CH), 74 (4 CH2), 118.7 (CH), 128 (2CH), 131.3, 142.9, 165.5 (2 C)

Example 5

Preparation of 4-azido-2,3,5,6-tetrafluoro-N-(3-hydroxypropyl)benzamide

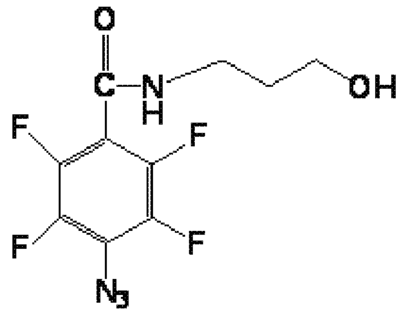

The synthesis followed the description of the process disclosed by "Facile UV Patterning of Robust Carbon Nanotube Forests Using Perfluoroarylazides"; S. J. Pastine, D. Okawa, B. Kessler, M. Rolandi, M. Llorente,A. Zettl, and J. M. J. Frechet; J. AM. CHEM. SOC. 2008, 130, 4238-4239.

In an two-necked flask were placed succinimidyl-(4-azido tetrafluoro)benzoate (204 mg, 0.614 mmol, 1.0 equiv.) and 2 mL of $CH_2Cl_2$. To the resulting solution was added proponolamine (60 $\mu$L, 0.737 mmol, 1.2 equiv.) via syringe.

After 2h, the reaction was diluted with $CH_2Cl_2$, washed with water (twice), brine, dried over magnesium sulfate, and concentrated in vacuo to give a off-white/pinkish solid in quantitative yield. The compound can be further purified via flash chromatography (hexanes/ethyl acetate = 1:2) to give a white solid.

**Melting point:** 95°C - 100°C

**IR** (NaCl): 3276 $cm^{-1}$ ;2133 $cm^{-1}$ ; 1656 $cm^{-1}$;

**H-NMR** ($CDCl_3$) (300 MHz): 6.6 (NH); 3.8 ($CH_2OH$); 3.6 ($CH_2NH$); 2.9 (OH); 1.9 $CH_2$);

**UV** (MeOH): 256 nm;

Example 6

Preparation of 4-azido-2,3,5,6-tetrafluoro-N-(4,4,5,5,6,6,7,7,8,8,9,9,10,10,11,11,11-heptadecafluoroundecyl) benzamide

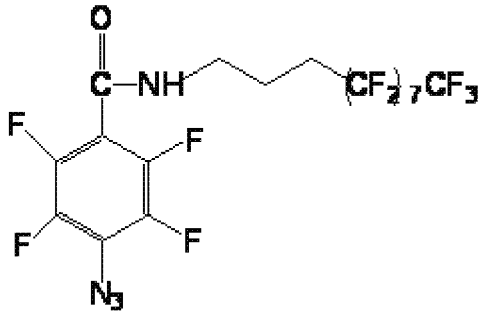

The synthesis followed the description of the process disclosed by "Facile UV Patterning of Robust Carbon Nanotube Forests Using Perfluoroarylazides"; S. J. Pastine, D. Okawa, B. Kessler, M. Rolandi, M. Llorente,A. Zettl, and J. M. J. Frechet; J. AM. CHEM. SOC. 2008, 130, 4238-4239.
In an two-necked flask were placed Succinimidyl-(4-azido tetrafluoro)benzoate (100 mg, 0.300 mmol, 1.0 equiv.), 2 mL of $CH_2Cl_2$, and 2 mL ethyl acetate. To the resulting solution was added 4,4,5,5,6,6,7,7,8,8,9,9,10,10,11,11,11-heptadecafluoroundecylamine (150 mg, 0.314 mmol, 1.05 equiv.) via syringe.
After 4h, the reaction suspension was directly loaded on to silica gel after flash chromatography (hexanes:ethyl acetate = 2:1 then 1:1) as a white solid.
**Melting point:** 100°C - 102°C
**IR** (NaCl) : 2127 $cm^{-1}$ ; 1654 $cm^{-1}$ ; 1147 $cm^{-1}$ ;
**H-NMR** ($CDCl_3$) (300 MHz): 6.1 (NH) ; 3.55($CH_2NH$);2.20 ($CH_2(CF_2)_7$);1.95 ($CH_2$-$CH_2$-$CH_2$);
**UV** (MeOH):256 nm;

Example 7

Preparation of 2-nitro-5-azidobenzoylglycine

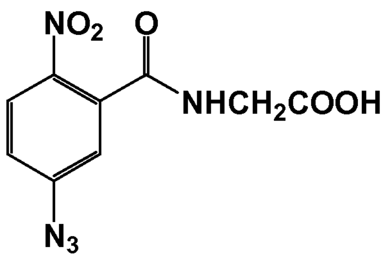

The synthesis followed the description of the process disclosed by "Photoaffinity Labeling of Peptide Hormone Binding Sites"; R. E. Galardy, L. C. Craig, J. D. Jamieson and M.P. Printz; The Journal of Biological Chemestry Vol. 249, No. 11, PP. 2510-2618, 1974.
To 2.5 g of sodium bicarbonate and 1.1 g of glycine in 70 ml of water was added 3.9 g of the N-hidroxysuccinimide ester of 4-azidobenzoic acid in 140 ml of dioxane. After 12 hour the mixture was rotary evaporated to 40 ml, cooled in ice, and adjusted to pH 2 with concentrated hydrochloric acid. All procedures were done in darkness. The solid product was recrystallized from water.
**Melting point:** 189°C - 195°C

**IR** (NaCl): 3287 $cm^{-1}$ ;2102 $cm^{-1}$ ; 1704 $cm^{-1}$ ; 1644 $cm^{-1}$; 1582 $cm^{-1}$;
**H-NMR** (DMSO) (300 MHz): 12.35 (OH); 9.0 (NH); 8.1,7.4,7.2 (aromatic H); 3.9 ($CH_2$);
**C-NMR** (DMSO): 170, 164 (C=O); 145,143,134,126,120,118 (aromatic H); 40 ($CH_2$);
**UV** (MeOH):307 nm;

Example 8

Preparation of N-(4,4,5,5,6,6,7,7,8,8,9,9,10,10,11,11,11-heptadecafluoroundecyl) 5-azido-2-nitrobenzoate

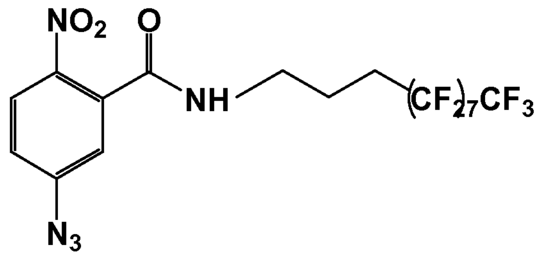

In an two-necked flask were placed N-Succinimidyl-5-azido-2-nitrobenzoate(100 m g) a n d 4,4,5,5,6,6,7,7,8,8,9,9,10,10,11,11,11-heptadecafluoro-undecylamine (180 mg) in 3 mL of ethyl acetate.
The solution was placed in darkness to stir overnight. The reaction product was directly loaded on to silica gel after flash chromatography (hexanes: ethyl acetate = 2:1) as a white solid.
**Melting point:** 105-110°C
IR (NaCl) : 3260 $cm^{-1}$; 2100 $cm^{-1}$; 1640 $cm^{-1}$; 1200 $cm^{-1}$; 1100 $cm^{-1}$;
**H-NMR** (DMSO) (300 MHz): 8.75 (NH); 8.1,7.4,7.25 (aromatic H); 2.45, 1.75 (aliphatic H);
**C-NMR** (DMSO): 165 (C=O); 145,142,135,125,120,118 (aromatic C); 40,30,20(aliphatic H);
**UV** (MeOH):308 nm;

Example 9

Preparation of N-(tris(hydroxymethyl)-5-azido-2-nitrobenzoate

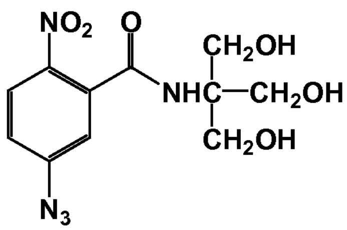

In an two-necked flask were placed N-Succinimidyl-5-azido-2-nitrobenzoate (100 mg) and 3,6 mL of dioxane. To the resulting solution was added a solution of tris(hydroxymethyl)aminomethane (80 mg in 1 mL of water) and 200 μL of TEA via syringe.
The solution was placed in darkness to stir overnight. The sample was brought to dry with a Rotavapor and vacuum pump (one night). The product was a yellow crystalline solid.
**Melting point:** 125-130°C
IR (NaCl) : 3381 $cm^{-1}$; 2124 $cm^{-1}$; 1713 $cm^{-1}$; 1583 cm-[1]; 1410 $cm^{-1}$;
**H-NMR** (DMSO) (300 MHz): 8.1 (NH); 7.4,7.1,7.05 (aromatic H); 3.75 (OH); 2.25(aliphatic H);
**C-NMR** (dmso): 165 (C=O); 145,140,135,127,125,120 (aromatic C); 62 (C-OH); 25,35,40(aliphatic C);
**UV** (MeOH):308 nm;

Example 10

Preparation of N-(Dodecaethylene glycol monomethyl ether)-5-azido-2-nitrobenzoate

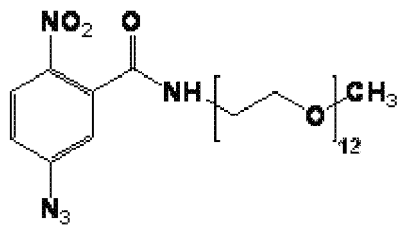

In an two-necked flask were placed N-Succinimidyl-5-azido-2-nitrobenzoate (100 mg), 5 mL of ethyl acetate and 200 μL of Triethylamine. To the resulting solution was added a solution of Methyl-$PEG_{12}$-Amine (purchased from ThermoFisher Scientific) in ethyl acetate (183 mg in 5 mL) via syringe. The solution was placed in darkness to stir for 16 hours. The solution (bright yellow) was extracted with 10 mL of brine for two times. The water phase was extracted with 10 mL of $CH_2Cl_2$ (3 times). The organic phase was dehydrated with anhydrous $MgSO_4$, filtered and brought to dry Rotavapor. The product is a dark yellow oil.
**IR** (NaCl) : 3200 $cm^{-1}$; 2873 $cm^{-1}$; 2121 $cm^{-1}$; 1669 $cm^{-1}$; 1104 $cm^{-1}$;
**H-NMR** ($CDCl_3$) (300 MHz): 6.8 (NH); 8.2,7.3,7.2 (aromatic H); 3.65 (aliphatic H); 3.3 ($OCH_3$);
**C-NMR** ($CDCl_3$): 165 (C=O); 146, 142, 135, 127, 120, 119 (aromatic C); 70 (aliphatic C); 57 ($OCH_3$);
**UV** (MeOH):305 nm;

Example 11

Determination of contact angle

As a reference, PA12 treated and non-treated surfaces have been tested for determining the contact angle; in particular:
i) a non-treated PA12 surface;
ii) a solvent treated PA12 surface;
iii) a 254 nm wavelength irradiated PA12 surface.
The solvent used for sample ii) was methanol. This comparative sample was tested in order to evaluate if the solvent is capable of influencing the surface wettability.
Each comparative sample (control sample) has been tested three times in three different positions (side, centre, side portions of the sample).
The results of the tests showing the measured contact angle (θ) are reported in the following Tables:

| **Non-treated PA12 surface (θ)** | | | | |
|---|---|---|---|---|
| | *test 1* | *test 2* | *test 3* | *media* |
| **Sample 1** | 85.6 | 86.4 | 89.3 | 87.1 |
| **Sample 2** | 87.0 | 88.5 | 89.8 | 88.4 |
| **Sample 3** | 85.0 | 86.6 | 85.8 | 85.8 |
| **Mean value** | 87.1 | | | |

| **Solvent-treated PA12 surface (θ)** | | | | |
|---|---|---|---|---|
| | *test 1* | *test 2* | *test 3* | *media* |
| **Sample 1** | 86.3 | 84.7 | 91.5 | 87.5 |
| **Sample 2** | 93.8 | 93.6 | 98.8 | 95.4 |

| | | | | |
|---|---|---|---|---|
| **Sample 3** | 88.6 | 94.1 | 82.0 | 88,3 |
| **Mean value** | 90.4 | | | |

| **Irradiated PA12 surface (θ)** | | | | |
|---|---|---|---|---|
| | *test 1* | *test 2* | *test 3* | *media* |
| **Sample 1** | 104,2 | 106,9 | 97,8 | 102,9 |
| **Sample 2** | 98, 9 | 96,3 | 91,0 | 95,4 |
| **Sample 3** | 104,9 | 107,5 | 100,26 | 104,2 |
| **Mean value** | 100.8 | | | |

The procedure above disclosed for the treatment of the polymeric surface was then carried out by using the polymeric material previously treated with the compound and method according to the invention disclosed above in order to enhance the hydrophilic character of the polymeric material surface. In particular, there have been used alcoholic solutions containing iv) N-(tris(hydroxymethyl)-4-azidobenzenesulphonylamide of Example 1, v) N(-2-hydroxyethyl)-4-azidobenzamide of Example 2 and vi) N-(2-hydroxyethyl)-4-azidobenzenesulphonylamide of Example 3, respectively. "CAM 200" KSV instrument and the software method of contact angle measurement were used.
The results are shown in the following Tables.
iv) N-(tris(hydroxymethyl)-4-azidobenzenesulphonylamide (of EXAMPLE 1)

Contact angle **(**θ**)** obtained by analysis with "CAM 200" instrument

| θ | | | | |
|---|---|---|---|---|
| | *test 1* | *test 2* | *test 3* | *media* |
| **Sample 1** | 53.33 | 50.02 | 57.49 | 53.6 |
| **Sample 2** | 50.24 | 49.84 | 55.42 | 51.8 |
| **Sample 3** | 59.31 | 51.01 | 43.30 | 51.3 |
| **Mean value** | 52.2 | | | |

v) N(-2-hydroxyethyl)-4-azidobenzamide (of EXAMPLE 2) Contact angle **(**θ**)** obtained by analysis with "CAM 200" instrument

| θ | | | | |
|---|---|---|---|---|
| | *test 1* | *test 2* | *test 3* | *media* |
| **Sample 1** | 50.98 | 51.48 | 52.43 | 51.63 |
| **Sample 2** | 59.78 | 67.55 | 64.69 | 64.01 |
| **Sample 3** | 60.54 | 62.95 | 61.38 | 61.62 |
| **Mean value** | 59.1 | | | |

vi) N-(2-hydroxyethyl)-4-azidobenzenesulphonyl-amide (of EXAMPLE 3)

Contact angle **(**θ**)** obtained by analysis with "CAM 200" instrument

| θ | | | | |
|---|---|---|---|---|
| | *test 1* | *test 2* | *test 3* | *media* |
| **Sample 1** | 47.93 | 48.24 | 54.51 | 50.2 |
| **Sample 2** | 56.60 | 53.09 | 49.96 | 53.2 |

| | | | | |
|---|---|---|---|---|
| **Sample 3** | 68.38 | 60.36 | 60.01 | 62.9 |
| **Mean value** | 55.4 | | | |

The above reported results show that the treatment of the PA12 surface with the azide compounds according to the present invention does actually modify the PA12 surface properties. In particular, it is apparent that the treatment according to the present invention has significantly increased the hydrophilicity of the PA12 surface as clearly shown by the relevant decrease of the contact angle of samples iv), v) and vi) with respect to comparative samples i), ii) and iii).

The procedure disclosed for the treatment of the polymeric surface was carried out also with a "G23" Kruss instrument. In particular, there have been used solutions containing iv) N-(tris(hydroxymethyl)-4-azidobenzenesulphonylamide of Example 1, vi) N-(2-hydroxyethyl)-4-azidobenzenesulphonylamide of Example 3, vii) N-(JEFFAMINE® M-600)-4-azidobenzamide of Example 4 and viii) N-(4,4,5,5,6,6,7,7,8,8,9,9,10,10,11,11,11-heptadecafluoro undecyl)-5-azido-2-nitrobenzoate of Example 8, respectively.

With the "G23" Kruss instrument the advancing contact angle was measured by depositing water droplets of 1-2 mm diameter.

The results are shown in the following Tables.

Contact angle (θ) obtained by analysis with "G23" Kruss instrument

| **Sample** | | θ |
|---|---|---|
| **1** | **PA12 (non-treated)** | 74±6 |
| **2** | **PA12 (UV processed)** | 80±5 |
| **iv** | **PA12+*N-(tris(hydroxymethyl)-4-azidobenzenesulphonylamide*** | 45±4 |
| **vi** | **PA12+*N-(2-hydroxyethyl)-4-azidobenzenesulphonylamide*** | 64±4 |
| **vii** | **PA12+*N-(Jeffamine(TM)M-600)-4-azidobenzamide*** | 58±4 |
| **viii** | **PA12+*N-(4,4,5,5,6,6,7,7,8,8,9,9,10,10,11,11,11-heptadeca fluoroundecyl-5-azido-2-nitrobenzoate*** | 92±5 |

Even with a different equipment and technique, the obtained results show that the azide compounds treatment according to the invention is able to modify the properties of the PA12 surface. In particular, it is evident that the surface treatments with iv, vi and vii decrease the value of contact angle with respect to the comparative samples 1 and 2. On the contrary, the surface treatment with viii increases the value of contact angle with respect to the comparative samples 1 and 2, thereby rendering the PA12 surface more hydrophobic.

The composition of the surface which underwent photoactivation has further been analysed by ESCA in order to determine the composition of the outer layers of the treated material for 8-10 nm depth and evaluating possible surface modifications. In particular, a Perkin Elmer PHI 5400 ESCA System has been used with a Mg anode X-ray source and a pressure within the analysis chamber of about $10^{-9}$ Torr. 1 cm samples were tested immediately after cutting and put into the chamber without any additional treatment.

For each sample, both the general spectra and C, N, O, F and S high resolution peaks have been acquired. The results are reported in the following Table.

Surface composition (atomic %) obtained by analysis XPS

| **Sample** | **O** | **C** | **N** | **S** | **F** |
|---|---|---|---|---|---|
| PA12 (theoretical composition) | 7.1 | 85.7 | 7.1 | - | - |
| PA12 (measured composition) | 13.1 | 80.0 | 3.4 | - | - |
| PA12 (UV processed) | 11.6 | 82.7 | 2.2 | 0.3 | - |
| PA12+N-(tris(hydroxymethyl)-4-azidobenzenesulphonylamide | 23.4 | 67.3 | 5.6 | 3.5 | - |
| PA12+N-(2-hydroxyethyl)-4-azidobenzenesulphonylamide | 20.6 | 66.5 | 7.3 | 4.7 | - |
| PA12+N-(Jeffamine(TM)M-600)-4-azidobenzamide | 17.7 | 76.1 | 3.8 | - | - |
| PA12+N-(4,4,5,5,6,6,7,7,8,8,9,9,10,10, 11,11,11-heptadeca fluoroundecyl-5-azido-2-nitrobenzoate | 9.0 | 78.5 | 4.8 | - | 6.6 |

From the above results, it appears that the treatment according to the invention increases both the N/C and the O/C rate. Figures 3-8 show the obtained spectra, wherein C1s peak is narrow for PA12 and PA12+UV due to the abundant C-C component present in PA12. For PA12+azide, the spectrum shows a visibly broader C1s peak and the C-O and C-N functionalities due to the presence of the azide on the modified surface.

EXAMPLE 12

Preparation of a catheter guide wire tube

A diluted solution of an azide compound of the present invention into a solvent (methanol) was prepared. The solvent can be selected from methanol, ethanol, acetone, acetonitrile, chloroform or mixture thereof. The solution was then introduced into a tube having inner diameter of 0.43 mm, external diameter of 0.60 mm and a length of 500 mm.

Two methods can be used for carrying out the surface modification according to the present invention.

The first is a static method according to which, after the tube has been filled with the above solution, firstly the tube ends are closed (e.g. welded), then the tube is placed into a dark chamber and irradiated for 1 hour with a 254 nm wavelength. The tube ends are successively opened and then the inner tube cavity is washed with water, methanol and acetone until no azide is detected into the wash water. The tube is then left drying in air. Alternatively, the second is a dynamic method according to which one end of the tube guide catheter is connected to a dark recipient containing the alcoholic solution of the azide. The device is placed inside a chamber provided with four lamps irradiating at a wavelength of 254 nm. Irradiation is performed for 1 hour while the alcoholic solution passes through the inside cavity of the tube. The thus treated inner surface is washed with water, methanol and acetone until no photoactivating compound is detected into the wash water. The tube is then left drying in air.

EXAMPLE 13

Extraction test

An extraction test was carried out in order to evaluate the force necessary for extracting a guide wire from a guide wire tube.

In particular, the extraction test was performed for evaluating the effect of the modification of the inner surface of a guide wire tube treated according to the present invention.
The guide wire tube inner surface was treated according to the teachings of Example 12 (dynamic method).
The guide wire and the guide wire tube were previously wetted with water. Then the guide wire was placed inside the guide wire tube so that the guide wire tip protruded of about 530 mm from the distal end of the guide wire tube. The guide wire tube and the guide wire were then introduced into a device provided with a tortuous path (so as to mimic a tortuous blood vessel) so that the guide wire exited of about 30 mm from the end portion of the tortuous path. The device was then clamped for ensuring blockage thereof. A dynamometer cell load was clamped to the guide wire tip exiting from the device and then the guide wire was extracted for a length of 850 mm at a speed of 60 mm/min. The extraction force was thus recorded.

The extraction test was performed for the guide wire tubes made from the following materials:

a) PA 12 (comparative)
b) Pebax 7233 (comparative)
c) trilayer made from PEBA - polyethylenic resin - HDPE (comparative)
d) P A 1 2 modified with N-(tris(hydroxymethyl))-4-azidobenzenesulphonilamide (invention)
e) PA12 modified with N-(2-hydroxyethyl)-4-azidobenzenesulphonylamide (invention)
f) PA12 modified with N-(2-hydroxyethyl)-4-azidobenzamide (invention)
g) Pebax 7233 modified with N-(tris(hydroxymethyl))-4-azidobenzenesulphonilamide (invention)
h) Pebax 7233 modified with N-(2-hydroxyethyl)-4-azidobenzenesulphonylamide (invention)
l) Pebax 7233 modified with N-(2-hydroxyethyl)-4-azidobenzamide (invention)

The guide wire tubes had all the following sizes:

inner diameter: 0.43 mm
external diameter: 0.60 mm
length: 500 mm

Guide wire tubes a), b) and from d) to 1) were made from a single polymeric layer, while only guide wire tube c) was a

tri-layered tube.
The tests were carried out by using two different types of guide wires.

In detail:

- Model 1: SKIPPER guide wire; manufactured by Brivant and commercialized by Invatec S.p.A.; usable length of 950 mm; Tip: floppy J; compliance: 0.014" (0,36 mm);
- Model 2: SKIPPER guide wire; manufactured by Brivant and commercialized by Invatec S.p.A.; usable length of 750 mm; Tip: floppy J; compliance: 0.014" (0,36 mm).

The results of the extraction tests are reported in the below Tables showing the forces (Newton) required for extracting the guide wire from the guide wire tube. Each force value (for each test) represents the mean value calculated in the 220-730 mm elongation range (where the elongation/force graph shows a substantially stable behaviour). Extraction tests carried out with guide wire Model 1:

| | |
|---|---|
| **Sample c) (Trilayered tube)** | |
| Test 1 | 12.38 N |
| Test 2 | 12.67 N |
| Test 3 | 12.30 N |
| | Mean value **12.45 N** |
| **Sample a) (non-modified PA12)** | |
| Test 1 | 21.25 N |
| Test 2 | 27.94 N |
| Test 3 | 26.40 N |
| Test 4 | 25.30 N |
| Test 5 | 31.61 N |
| Test 6 | 27.27 N |
| Test 7 | 27.63 N |
| | Mean value **26.77 N** |
| **Sample d) (modified PA12-N-(tris(hydroxymethyl))-4-azidobenzenesulphonylamide)** | |
| Test 1 | 16.43 N |
| Test 2 | 16.20 N |
| Test 3 | 17.84 N |
| Test 4 | 15.60 N |
| Test 5 | 20.45 N |
| Test 6 | 16.72 N |
| Test 7 | 18.25 N |
| Test 8 | 19.96 N |
| | Mean value **17.68 N** |
| **Sample e) (modified PA12 with N-(2-hydroxyethyl)-4-azidobenzenesulphonylamide)** | |
| Test 1 | 22.81 N |
| Test 2 | 22.37 N |
| Test 3 | 20.63 N |
| | Mean value **21.94 N** |

| **Sample g) (modified PA12 with N-(2-hydroxyethyl)-4-azidobenzamide)** | |
|---|---|
| Test 1 | 22.93 N |
| Test 2 | 24.19 N |

(continued)

| Sample g) (modified PA12 with N-(2-hydroxyethyl)-4-azidobenzamide) | |
|---|---|
| | Mean value **23.56 N** |

- Extraction tests carried out with guide wire Model 2:

| Sample c) (Trilayered tube) | |
|---|---|
| Test 1 | 4.47 N |
| Test 2 | 4.51 N |
| Test 3 | 4.15 N |
| | Mean value **4.38 N** |
| **Sample b) (non-modified PEBAX 7233)** | |
| Test 1 | 4.88 N |
| Test 2 | 4.97 N |
| | Mean value **4.93 N** |
| **Sample g) (modified PEBAX with N-(tris(hydroxymethyl))-4-azidobenzenesulphonylamide)** | |
| Test 1 | 4.02 N |
| Test 2 | 3.71 N |
| Test 3 | 3.51 N |
| | Mean value **3.75 N** |
| **Sample h) (modified PEBAX with N-(2-hydroxyethyl)-4-azidobenzenesulphonylamide)** | |
| Test 1 | 3.31 N |
| Test 2 | 3.50 N |
| | Mean value **3.41 N** |
| **Sample 1) (modified PEBAX with N-(2-hydroxyethyl)-4-azidobenzamide)** | |
| Test 1 | 5.20 N |
| Test 2 | 3.76 N |
| | Mean value **4.48 N** |

As per the above results, it can be seen that the modification of the inner surface of a guide wire tube generally contributes in decreasing the force required for extracting the guide wire from the guide wire tube.
In detail, as per the above results, it can be seen that the modification of the inner surface of a PEBAX guide wire tube allows a lower force to be required for extracting the guide wire (Model 2). Moreover, the modification with N-(tris(hydroxymethyl))-4-azidobenzenesulphonylamide and N-(2-hydroxyethyl)-4-azidobenzenesulphonylamide advantageously leads to a performance which is even better than the performance of the comparative tri-layered guide wire tube.

Therefore, the above results clearly show that the photoactivation reaction with the above compounds according to the present invention remarkably contributes in reducing the force required for the extraction of a guide wire from a guide wire tube with respect to a non-treated PA12 guide wire tube.

EXAMPLE 14

Evaluation of the photoactivation reaction yield of the polymeric surface

This analysis shows how the photoactivation reaction yield of the polymeric surface is influenced by the substituted aromatic azide used for the reaction.

The proposed reaction mechanism for light excitation of arylazides is summarized in Fig. 9 ("Thrombin inhibitors grafting on polyester membranes for the preparation of blood-compatible materials", Claudio Salvagnini, 2005).
Firstly arylazide (1) undergoes loss of molecular nitrogen and transient formation of singlet arylnitrene (2S). This unstable compound may rapidly react by insertion on hydrocarbons (3) or by internal rearrangement to 1,2-azacycloheptatetraene (4). This is an electrondeficient species and it reacts predominantly with nucleophiles (Nu), forming azepine adducts (5). Small amount of triplet nitrene species (2T) is formed through a process of intersystem crossing. Triplet nitrene is essentially a diradical species that is capable of hydrogen-radical abstraction and covalent binding to hydrocarbon substrates (7).

For the treatment of the polymeric surface according to the invention, in order to enhance the hydrophilic or hydrophobic character of the polymeric material surface, and in particular for a good yield of the photoactivation reaction, it is important that most part of the arylazide in the solution reacts by covalent bindings to hydrocarbon substrates (3) or (7). In particular, for a good yield of the photoactivation reaction, it is important that only a residual part of the arylazide in the solution reacts by forming azepine adducts (5), which represents a waste of the reaction.

Coming back to the reaction, once formed the singlet arylnitrene (2S) usually undergoes intersystem crossing to the corresponding triplets at rates that depend on the nature of the nitrene. For covalent bond formation, singlet arylnitrene is better since it acquires an electron pair from its substrate in a single reaction step. The electronic nature of triplet nitrene (2T), on the contrary, forces it to undergo two-steps reactions in which a covalent bond between the nitrene and its substrate is often made only in the second step. For this reason, usually in the reaction mechanism for light excitation of arylazides, the azepine formation is the major reaction path while only a small part of the singlet arylnitrene (2s) is slowly converted into hydrocarbon substrates (3) or into triplet nitrene (2T).

As anticipated above, for a good yield of the photoactivation reaction it is important that most part of the arylazide in the solution reacts by covalent bindings with hydrocarbon substrates (3) or (7). Compounds [4-azido-2,3,5,6-tetrafluoro-N-(3-hydroxypropyl)benzamide of Example 5; 4-azido-2,3,5,6-tetrafluoro-N-(4,4,5,5,6,6,7,7,8,8,9,9,10,10,11,11,11-heptadecafluoroundecyl) benzamide of Example 6; 2-nitro-5-azidobenzoylglycine of Example 7; N-(4,4,5,5,6,6,7,7,8,8,9,9,10,10,11,11,11-heptadecafluoroundecyl) 5-azido-2-nitrobenzo a t e of Example 8; N-(tris(hydroxymethyl)-5-azido-2-nitrobenzoate of Example 9 and N-(Dodecaethylene glycol monomethyl ether)-5-azido-2-nitrobenzoate of Example 10 have shown to provide good reaction rate and yield.

The photochemical reactions of these substituted aromatic azides have been studied by using UV irradiation light. Two different samples have been prepared with:

- 3 ml of a solution $10^{-2}$ M of N-(2-hydroxyethyl)-4-azidobenzenesulphonylamide of Example 3 (Az1);
- 3 ml of a solution $10^{-2}$ M of 4-azido-2,3,5,6-tetrafluoro-N-(3-hydroxypropyl)benzamide of Example 5 (Az2).

The solvent used for the samples was methanol. A PA12 surface has been immersed in each solution, and the samples have been irradiated with a light wavelength of 254 nm.

These samples were tested at different times with a TLC test (eluent: 100% of ethyl acetate) in order to evaluate the yield of the photochemical reaction. The TLC results of the photochemical reaction, provided at different times and for both the samples Az1 and Az2, are shown in Figure 10.

Time t=0

The presence of the azide is indicated by a spot and correlated to a specific distance Rf, where

$$R_f = \frac{\text{distance travelled by the compound}}{\text{distance travelled by the solvent font}}$$

At the starting point, it is possible to see the presence of two spots which represent the two azides, and in particular the presence of arylazide available for the reaction with the polymeric surface. The specific starting values of $R_f$ are: $R_{fAz1}$=0.64 for AZ1 and $R_{fAz2}$=0.58 for AZ2.

Time t=5 minutes

After a certain reaction period (5 minutes) it is possible to detect also the spots corresponding to the products of the two reactions, indicated as Ph1 and Ph2 respectively.
In particular, as regards the reaction concerning the N-(2-hydroxyethyl)-4-azidobenzenesulphonylamide, it is possible to see:

- a very small spot (as compared to the spot of t=0) corresponding to a small amount of arylnitrene available for the reaction and indicated by the same $R_{fAz1}$=0.64;
- a new spot, corresponding to a small amount of azepine, indicated by $R_{fPh1}$=0.46. This situation indicates that part of arylnitrene has been transformed into azepine.

As regards the reaction concerning the 4-azido-2,3,5,6-tetrafluoro-N-(3-hydroxypropyl)benzamide, it is possible to see only a small spot (as compared to the spot of t=0) corresponding to a small amount of arylazide available for the reaction and indicated by the same $R_{fAz2}$=0.58. In this situation, the absence of azepine (i.e. the absence of the relative spot) and the decrease in the amount of arylazide available for the reaction (i.e. represented by a small relative spot) indicates that part of arylnitrene has reacted with the polymeric surface.

Time t=30 minutes

At this stage of the reaction, in considering the N-(2-hydroxyethyl)-4-azidobenzenesulphonylamide, it is still possible to see both of the spots: the spot corresponding to arylazide ($R_{fAz1}$=0.64) (which is further decreased) and the spot corresponding to azepine ($R_{fPh1}$=0.46) (which is increased). As regards the reaction concerning the 4-azido-2,3,5,6-tetrafluoro-N-(3-hydroxypropyl)benzamide, it is now possible to see:

- the small spot corresponding to arylazide available for the reaction and indicated by the same $R_{fAz2}$=0.58;
- a new spot, corresponding to a small amount of azepine, indicated by $R_{fPh2}$=0.35. This situation indicates that only now part of arylnitrene has been transformed in azepine.

Time t=1 hour and 30 minutes

At this stage of the reaction, in considering the N-(2-hydroxyethyl)-4-azidobenzenesulphonylamide, it is possible to see only the spot corresponding to azepine ($R_{fPh1}$=0.46). This situation indicates that there is no more arylnitrene available for the reaction with the polymeric surface and thus the reaction is terminated.
As regards the reaction concerning the 4-azido-2,3,5,6-tetrafluoro-N-(3-hydroxypropyl)benzamide, it is still possible to see both of the spots: the small spot corresponding to arylazide available for the reaction and the spot corresponding to azepine. This situation indicates that there is still arylnitrene available for the reaction with the polymeric surface and thus the reaction is still on-going.

The TLC measures obtained for the azide with electron-withdrawing substituents shows that the azepine, which represents a reaction waste, does not appear immediately. The above results show that the azide enhanced with electron-withdrawing substituents (for example -F and $-NO_2$) have a good yield of the photochemical reaction with the polymeric surface.

As mentioned above, according to a further object of the present invention, the disclosed photoactivated polymers can be used for the preparation of medical devices such as, for instance, balloon catheters, stent delivery systems, clot extraction catheters, multipurpose catheters.

For instance, in case a balloon catheter is considered, by treating the inner surface of the catheter inflation tube according to the present invention, the inflation time and especially the deflation time of the balloon can be favourably improved (i.e. decreased). In fact, thanks to treated inner surface of the present invention, a reduced friction between the inflation fluid and the inner surface of the balloon inflation tube is obtained, thereby resulting in a very easy and quick sliding of the inflation fluid along the inflation tube.

According to the present invention, the disclosed para-substituted arylazide compounds are used in order to modify the properties of a polymeric inner surface of a catheter tube. In order to reduce the friction coefficient, generally the catheter tubes produced according to the invention are provided with a hydrophilic inner surface. However, thanks to the above treatment method, the inner surface may be rendered either more hydrophilic or more hydrophobic according to the need by changing the nature of the para substitutent in the arylazide compound.

The thus treated inner surface has surprisingly found not to be weakened with respect to the polymeric surfaces treated according to the methods known in the art.

In fact, the Applicant has found that a catheter tube treated according to the present invention advantageously has a reduced thickness with respect to a tri-layered catheter tube known in the art, without the mechanical resistance of the catheter tube being negatively affected.

In the particular case wherein a balloon catheter is considered, the method of the invention allows: a) to decrease the outer diameter of the tube (guide wire tube), thereby increasing the cross-section area dedicated to the passage of the inflation fluid, and/or b) to increase the inner diameter of the tube (guide wire tube) without modifying the outer diameter thereof, thereby providing a larger passageway for the guide wire and reducing the friction risks between the guide wire and the tube (guide wire tube).

In case a clot extraction catheter is concerned, the reduced thickness of the tube allows either to reduce the external diameter of the catheter (and thus the radial dimension thereof) or to increase the aspiration area and efficiency without modifying the catheter outer diameter.

## Claims

1. A medical device comprising a catheter tube having a hollow body made of a polymeric material, said body having a surface, **characterised in that** said surface comprises covalently bound groups of formula

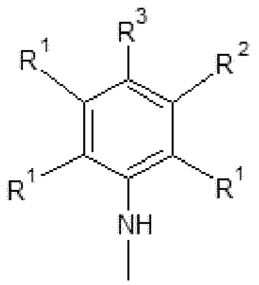

wherein $R^1$ and $R^2$ independently from each other are H or F, and $R^3$ is a Z group; or
wherein each $R^1$ independently is H or F, $R^2$ is a Z group, and $R^3$ is $-NO_2$;
wherein the Z group is selected from $-C(O)NH-R_a$, $-S(O)_2NH-R_a$ and $-P(O)_2NH-R_a$ wherein $R_a$ is $C_1$-$C_4$ linear or branched saturated alkyl chain optionally substituted with one or more polar functional groups selected from -OH, -COOH, $-SO_3$, $-PO_4$, $-NH_2$, $-NH_4+$ or with a $-(CF_2)_m-CF_3$ perfluoroalkyl group wherein m is 1 to 70; or wherein $R_a$ is $-(CHRCH_2O)_n-X$ wherein n is 1 to 70, R is H or $-CH_3$ and X is selected from H, saturated branched or linear $C_1$-$C_4$ alkyl chain or a $-(CH_2)_p-O-(CH_2)_q-W$ group wherein W is H, $-CH_3$ or $-NH_2$ and wherein p and q are independently 1 to 30; or wherein $R_a$ is a $C_1$-$C_{70}$ linear or branched saturated alkyl chain or an aromatic group.

2. The medical device according to claim 1, wherein the linear or branched saturated alkyl chain within $R_a$ is selected from the group comprising polyethylene, polypropylene and polyolefins.

3. The medical device according to any one of claims 1 or 2, wherein the aromatic group of $R_a$ is selected from the group comprising xylene, polystyrene and acrylonitrile butadiene styrene, and/or wherein the perfluoroalkyl group within $R_a$ is of formula $-(CF_2)_nCF_3$ wherein n= is from 1 to 70, and/or wherein the perfluoroalkyl group is polytetrafluoroethylene.

4. The medical device according to any one of the preceding claims, wherein within the Z group, $R_a$ is selected from $-(CH_2CH_2)OH$, $-(CH_2CH_2CH_2)OH$, $-C(CH_2OH)_3$, $-(CH(CH_3)CH_2O)_9-CH_2CH_2OCH_3$, $-(CH_2)(CF_2)_7CF_3$.

5. The medical device according to any one of the preceding claims, wherein the polymeric material is selected in the group comprising the polyamide-based copolymers of general formula

$$H-(O-PF-OOC-PA-COO-PF-OOC-PA-CO)_n-OH$$

wherein PA is a polyamide segment and PF is a diol segment comprising OH-terminating dimer diol polyesters and n is between 5 and 20 and an elastomer obtained by the polymerization of a polyamide forming block compound selected in the group comprising an aminocarboxylic acid of formula (1) below and a lactam of formula (2) below

$$H_2N-R^1-COOH \qquad (1)$$

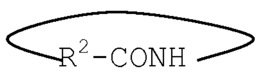 (2)

with a polyetherdiaminic triblock of formula (3) below:

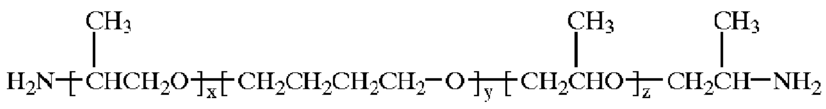 (3)

and a dicarboxylic acid of formula (4) below:

$$HOOC\text{-}(R^3)_m\text{-}COOH \qquad (4)$$

wherein $R^1$, $R^2$ and $R^3$ are each binding groups comprising a hydrocarbon chain therein, which may be interrupted by one or more amide groups and wherein R1 and R2 comprise independently an alkylene group having 2 to 20 carbon atoms and amide bonds and R3 comprises an alkylene group having 1 to 20 carbon atoms and wherein x may change from 1 to 20, y may change from 4 to 50, z may change from 1 to 20 and wherein m is 0 or 1.

**6.** The medical device according to any one of the preceding claims, wherein the catheter tube is a guide wire tube or an inflation tube and in which said surface comprises hydrophilic groups, and/or wherein the catheter tube is a guide wire tube or an aspiration tube and in which said surface comprises hydrophobic groups, and/or wherein the catheter tube surface is the inner surface, and/or wherein the catheter tube surface is the outer surface, and/or wherein the catheter tube surface is the inner and the outer surface.

**7.** A method for modifying the surface of the medical device according to any one of the preceding claims, said method comprising the steps of:

a) preparing a solution of a compound of formula (I)

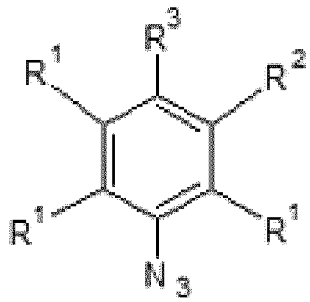

wherein $R^1$ and $R^2$ independently from each other are H or F, and $R^3$ is a Z group; or wherein each $R^1$ is independently H or F, $R^2$ is a Z group, and $R_3$ is $-NO_2$;
wherein the Z group is selected from $-C(O)NH-R_a$, $-S(O)_2NH-R_a$ and $-P(O)_2NH-R_a$ wherein $R_a$ is $C_1$-$C_4$ linear or branched saturated alkyl chain optionally substituted with one or more polar functional groups selected from -OH, -COOH, $-SO_3$, $-PO_4$, $-NH_2$, $-NH_4$+ or with a $-(CF_2)_m-CF_3$ perfluoroalkyl group wherein m is 1 to 70; or wherein $R_a$ is $-(CHRCH_2O)_n-X$ wherein n is 1 to 70, R is H or $-CH_3$ and X is selected from H, saturated branched or linear $C_1$-$C_4$ alkyl chain or a $-(CH_2)_p-O-(CH_2)_q-W$ group wherein W is H, $-CH_3$ or $-NH_2$ and wherein p and q are independently 1 to 30; or wherein $R_a$ is a $C_1$-$C_{70}$ linear or branched saturated alkyl chain or an aromatic group; in a suitable solvent;

b) contacting the surface of the medical device with the solution prepared from step a);
c) irradiating the surface obtained from step b) with a radiation capable of photoactivating said surface.

**8.** The method according to claim 7, wherein the compound of formula (I) is selected in the group comprising: N-(tris(hydroxymethyl)-4-azidobenzenesulphonylamide, N(-2-hydroxyethyl)-4-azidobenzamide, N-(2-hydroxyethyl)-4-azido-benzene sulphonylamide and

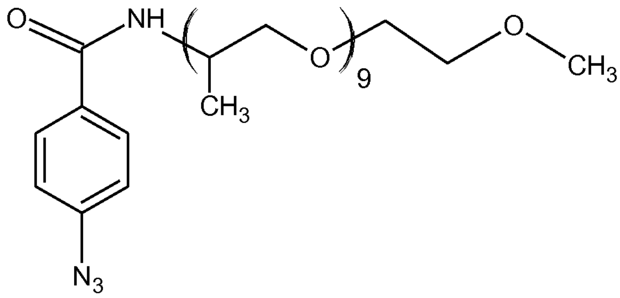

,

4-azido-2,3,5,6-tetrafluoro-N-(3-hydroxypropyl)benzamide, 4-azido-2,3,5,6-tetrafluoro-N-(4,4,5,5,6,6,7,7,8,8,9,9,10,10,11,11,11-heptadeca fluoroundecyl) benzamide, 2-nitro-5-azidobenzoylglycine, N-(4,4,5,5,6,6,7,7,8,8,9,9,10,10,11,11,11-heptadeca fluoroundecyl)5-azido-2-nitrobenzoate, N-(tris(hydroxymethyl)-5-azido-2-nitrobenzoate, N-(Dodecaethylene glycol monomethyl ether)-5-azido-2-nitrobenzoate.

**9.** The method according to any one of claims 7 or 8, wherein the solvent of step a) is selected from methanol, ethanol, acetonitrile and chloroform, and/or wherein said radiation has a wavelength capable of passing through the polymeric material of the medical device, and/or wherein said radiation has a wavelength comprised from 200 nm to 600 nm, preferably from 250 nm to 350 and more preferably from 230 nm to 300 nm, and/or wherein the irradiation of step c) is performed in a dark environment at room temperature, and/or wherein the irradiation of step c) is performed for 0.5-1 hour.

**10.** The method according to any one of claims 7 to 9, wherein step b) is performed by flowing the solution comprising the compound of formula (I) through the surface of the medical device, and/or comprising, before step b), a step wherein the surface to be treated is washed and dried, and/or wherein after step b), the surface is washed in order to remove the unbound compounds of formula (I), and/or wherein said surface of the medical device is the inner surface, the outer surface or both.

**11.** A compound of formula (I) which is N-(tris(hydroxymethyl)-4-azidobenzenesulphonylamide, N-(2-hydroxyethyl)-4-azidobenzenesulphonylamide,

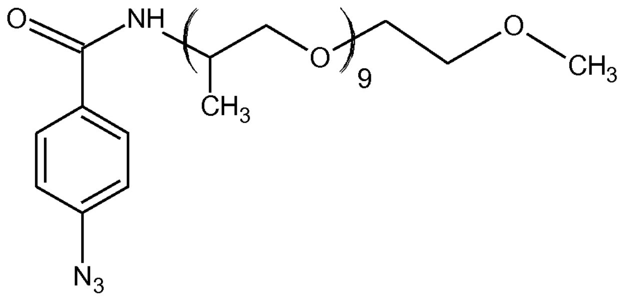

,

N-(4,4,5,5,6,6,7,7,8,8,9,9,10,10,11,11,11-heptadecafluoroundecyl) 5-azido-2-nitrobenzoate, N-(tris(hydroxymethyl)-5-azido-2-nitrobenzoate or N-(dodecaethylene glycol monomethyl ether)-5-azido-2-nitrobenzoate.

**12.** The use of a compound of claim 11 for modifying the properties of a polymeric surface.

**13.** The use of a compound according to claim 12, wherein said polymeric surface is the surface of a medical device, and/or wherein said surface is the inner and/or the outer surface of a medical device.

**14.** The use of a compound according to any one of claims 12 to 13, wherein said surface is the inner surface of a balloon of a catheter; or wherein said surface is the inner and/or outer surface of a catheter tube wherein typically said catheter tube is selected from a guide wire tube, an inflation tube and an aspiration tube.

## Patentansprüche

1. Medizinische Vorrichtung, umfassend einen Katheterschlauch, der einen aus einem Polymermaterial gefertigten hohlen Körper aufweist, wobei der Körper eine Oberfläche aufweist, **dadurch gekennzeichnet, dass** die Oberfläche kovalent gebundene Gruppen der folgenden Formel umfasst:

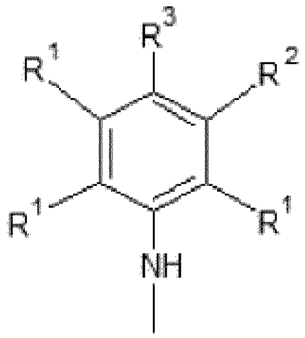

wobei $R^1$ und $R^2$ unabhängig voneinander H oder F sind und $R^3$ eine Gruppe Z ist; oder
wobei jedes $R^1$ unabhängig H oder F ist, $R^2$ eine Gruppe Z ist und $R^3$ $-NO_2$ ist;
wobei die Gruppe Z ausgewählt ist aus $-C(O)NH-R_a$, $-S(O)_2NH-R_a$ und $-P(O)_2NH-R_a$, wobei $R_a$ eine lineare oder verzweigte, gesättigte $C_1$-$C_4$-Alkylkette ist, wahlweise substituiert mit einer oder mehreren polaren funktionellen Gruppen ausgewählt aus -OH, -COOH, $-SO_3$, $-PO_4$, $-NH_2$, $-NH_4$+ oder mit einer $-(CF_2)_m-CF_3$-Perfluoralkylgruppe, wobei m 1 bis 70 ist; oder wobei $R_a$ $-(CHRCH_2O)_n-X$ ist, wobei n 1 bis 70 ist, R H oder $-CH_3$ ist und X aus H, einer gesättigten, verzweigten oder linearen $C_1$-$C_4$-Alkylkette oder einer $-(CH_2)_p-O-(CH_2)_q-W$-Gruppe, wobei W H, $-CH_3$ oder $-NH_2$ ist und wobei p und q unabhängig 1 bis 30 sind, ausgewählt ist; oder wobei $R_a$ eine lineare oder verzweigte, gesättigte $C_1$-$C_{70}$-Alkylkette oder eine aromatische Gruppe ist.

2. Medizinische Vorrichtung nach Anspruch 1, wobei die lineare oder verzweigte, gesättigte Alkylkette innerhalb von $R_a$ aus der Gruppe umfassend Polyethylen, Polypropylen und Polyolefine ausgewählt ist.

3. Medizinische Vorrichtung nach einem der Ansprüche 1 oder 2,
wobei die aromatische Gruppe von $R_a$ aus der Gruppe umfassend Xylol, Polystyrol und Acrylnitril-Butadien-Styrol ausgewählt ist, und/oder wobei die Perfluoralkylgruppe innerhalb von $R_a$ die Formel $-(CF_2)_nCF_3$ aufweist, wobei n von 1 bis 70 ist, und/oder wobei die Perfluoralkylgruppe Polytetrafluorethylen ist.

4. Medizinische Vorrichtung nach einem der vorhergehenden Ansprüche, wobei innerhalb der Gruppe Z $R_a$ aus $-(CH_2CH_2)OH$,$-(CH_2CH_2CH_2)OH$, $-C(CH_2OH)_3$, $-(CH(CH_3)CH_2O)_9-CH_2CH_2OCH_3$,$-(CH_2)(CF_2)_7CF_3$ ausgewählt ist.

5. Medizinische Vorrichtung nach einem der vorhergehenden Ansprüche, wobei das Polymermaterial aus der Gruppe umfassend die Copolymere auf Polyamidbasis der allgemeinen Formel

$$H-(O-PF-OOC-PA-COO-PF-OOC-PA-CO)_n-OH$$

ausgewählt ist, wobei PA ein Polyamidsegment ist und PF ein Diolsegment ist, das OH-terminierende Dimerdiolpolyester umfasst, und n zwischen 5 und 20 ist und ein Elastomer, das durch die Polymerisation einer polyamidbildenden Blockverbindung, die aus der Gruppe umfassend eine Aminocarbonsäure der nachstehenden Formel (1) und ein Lactam der nachstehenden Formel (2)

$$H_2N-R^1-COOH \qquad (1)$$

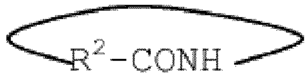 (2)

ausgewählt ist,
mit einem polyetherdiaminischen Triblock der nachstehenden Formel (3):

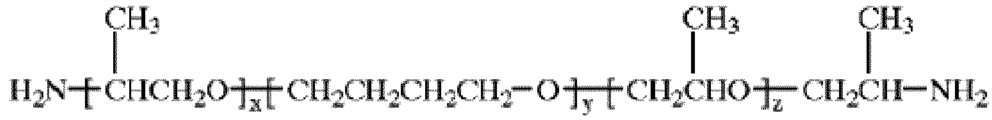

(3)

und einer Dicarbonsäure der nachstehenden Formel (4):

$$HOOC\text{-}(R^3)_m\text{-}COOH \qquad (4)$$

erhalten wird,
wobei $R^1$, $R^2$ und $R^3$ jeweils Bindungsgruppen sind, die eine Kohlenwasserstoffkette darin umfassen, die durch eine oder mehrere Amidgruppen unterbrochen sein kann und wobei R1 und R2 unabhängig eine Alkylengruppe mit 2 bis 20 Kohlenstoffatomen und Amidbindungen umfassen und R3 eine Alkylengruppe mit 1 bis 20 Kohlenstoffatomen umfasst und wobei x sich von 1 bis 20 ändern kann, y sich von 4 bis 50 ändern kann, z sich von 1 bis 20 ändern kann und wobei m 0 oder 1 ist.

**6.** Medizinische Vorrichtung nach einem der vorhergehenden Ansprüche, wobei der Katheterschlauch ein Führungsdrahtschlauch oder ein Aufblasschlauch ist und in dem die Oberfläche hydrophile Gruppen umfasst und/oder wobei der Katheterschlauch ein Führungsdrahtschlauch oder ein Absaugschlauch ist und in dem die Oberfläche hydrophobe Gruppen umfasst und/oder wobei die Katheterschlauchoberfläche die Innenfläche ist und/oder wobei die Katheterschlauchoberfläche die Außenfläche ist und/oder wobei die Katheterschlauchoberfläche die Innen- und die Außenfläche ist.

**7.** Verfahren zum Modifizieren der Oberfläche der medizinischen Vorrichtung nach einem der vorhergehenden Ansprüche, wobei das Verfahren die folgenden Schritte umfasst:

a) Herstellen einer Lösung einer Verbindung der Formel (I)

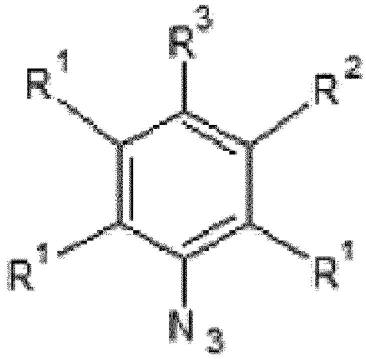

wobei $R^1$ und $R^2$ unabhängig voneinander H oder F sind und $R^3$ eine Gruppe Z ist; oder
wobei jedes $R^1$ unabhängig H oder F ist, $R^2$ eine Gruppe Z ist und $R_3$ $-NO_2$ ist;
wobei die Gruppe Z ausgewählt ist aus $-C(O)NH-R_a$, $-S(O)_2NH-R_a$ und $-P(O)_2NH-R_a$, wobei $R_a$ eine lineare oder verzweigte, gesättigte $C_1$-$C_4$-Alkylkette ist, wahlweise substituiert mit einer oder mehreren polaren funktionellen Gruppen ausgewählt aus -OH, -COOH, $-SO_3$, $-PO_4$, $-NH_2$, $-NH_4$+ oder mit einer $-(CF_2)_m-CF_3$-Perfluoralkylgruppe, wobei m 1 bis 70 ist; oder wobei $R_a$ $-(CHRCH_2O)_n-X$ ist, wobei n 1 bis 70 ist, R H oder $-CH_3$ ist und X aus H, einer gesättigten, verzweigten oder linearen $C_1$-$C_4$-Alkylkette oder einer $-(CH_2)_p-O-(CH_2)_q-W$-Gruppe, wobei W H, $-CH_3$ oder $-NH_2$ ist und wobei p und q unabhängig 1 bis 30 sind, ausgewählt ist; oder wobei $R_a$ eine lineare oder verzweigte, gesättigte $C_1$-$C_{70}$-Alkylkette oder eine aromatische Gruppe ist, in einem geeigneten Lösungsmittel;

b) Inkontaktbringen der Oberfläche der medizinischen Vorrichtung mit der in Schritt a) hergestellten Lösung;
c) Bestrahlen der in Schritt b) erhaltenen Oberfläche mit einer Strahlung, die in der Lage ist, die Oberfläche mit Licht zu aktivieren.

**8.** Verfahren nach Anspruch 7, wobei die Verbindung der Formel (I) ausgewählt ist aus der Gruppe umfassend: N-(Tris(hydroxymethyl)-4-azidobenzolsulfonylamid, N(-2-Hydroxyethyl)-4-azidobenzamid, N-(2-Hydroxyethyl)-4-

azidobenzolsulfonylamid und

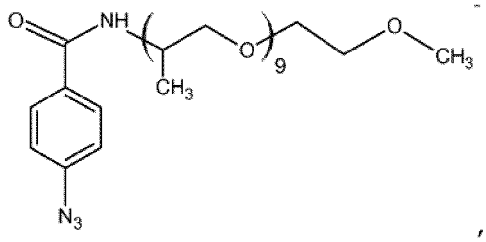

,

4-Azido-2,3,5,6-tetrafluor-N-(3-hydroxypropyl)benzamid, 4-Azido-2,3,5,6-tetrafluor-N-(4,4,5,5,6,6,7,7,8,8,9,9,10,10,11,11,11-heptadecafluorundecyl)benzamid, 2-Nitro-5-azidobenzoylglycin, N-(4,4,5,5,6,6,7,7,8,8,9,9,10,10,11,11,11-heptadecafluorundecyl)-5-azido-2-nitrobenzoat, N-(Tris(hydroxymethyl)-5-azido-2-nitrobenzoat, N-(Dodecaethylenglykolmonomethylether)-5-azido-2-nitrobenzoat.

**9.** Verfahren nach einem der Ansprüche 7 oder 8, wobei das Lösungsmittel aus Schritt a) aus Methanol, Ethanol, Acetonitril und Chloroform ausgewählt ist und/oder wobei die Strahlung eine Wellenlänge aufweist, die durch das Polymermaterial der medizinischen Vorrichtung hindurchgehen kann und/oder wobei die Strahlung eine Wellenlänge von 200 nm bis 600 nm, vorzugsweise von 250 nm bis 350 nm und mehr bevorzugt von 230 nm bis 300 nm aufweist und/oder wobei die Bestrahlung von Schritt c) in einer dunklen Umgebung bei Raumtemperatur durchgeführt wird und/oder wobei die Bestrahlung von Schritt c) 0,5-1 Stunde lang durchgeführt wird.

**10.** Verfahren nach einem der Ansprüche 7 bis 9, wobei der Schritt b) durchgeführt wird, indem die Lösung, die die Verbindung der Formel (I) umfasst, durch die Oberfläche der medizinischen Vorrichtung fließen gelassen wird und/oder umfassend vor Schritt b) einen Schritt, wobei die zu behandelnde Oberfläche gewaschen und getrocknet wird und/oder wobei nach Schritt b) die Oberfläche gewaschen wird, um die nicht gebundenen Verbindungen der Formel (I) zu entfernen und/oder wobei die Oberfläche der medizinischen Vorrichtung die Innenfläche, die Außenfläche oder beides ist.

**11.** Verbindung der Formel (I), die N-(Tris(hydroxymethyl)-4-azidobenzolsulfonylamid, N-(2-Hydroxyethyl)-4-azidobenzolsulfonylamid,

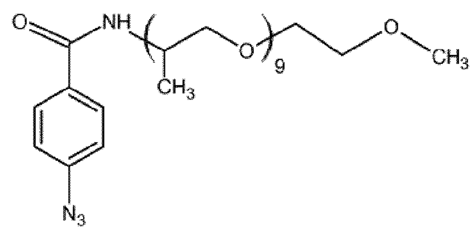

,

N-(4,4,5,5,6,6,7,7,8,8,9,9,10,10,11,11,11-heptadecafluorundecyl)-5-azido-2-nitrobenzoat, N-(Tris(hydroxymethyl)-5-azido-2-nitrobenzoat oder N-(Dodecaethylenglykolmonomethylether)-5-azido-2-nitrobenzoat ist.

**12.** Verwendung einer Verbindung nach Anspruch 11 zum Modifizieren der Eigenschaften einer Polymeroberfläche.

**13.** Verwendung einer Verbindung nach Anspruch 12, wobei die Polymeroberfläche die Oberfläche einer medizinischen Vorrichtung ist und/oder wobei die Oberfläche die Innen- und/oder die Außenfläche einer medizinischen Vorrichtung ist.

**14.** Verwendung einer Verbindung nach einem der Ansprüche 12 bis 13, wobei die Oberfläche die Innenfläche eines Ballons eines Katheters ist; oder wobei die Oberfläche die Innen- und/oder die Außenfläche eines Katheterschlauchs ist, wobei typischerweise der Katheterschlauch aus einem Führungsdrahtschlauch, einem Aufblasschlauch und einem Absaugschlauch ausgewählt ist.

## Revendications

1. Dispositif médical comprenant un tube de cathéter ayant un corps creux fabriqué en un matériau polymère, ledit corps ayant une surface, **caractérisé en ce que** la surface comprend des groupes liés de façon covalente de formule

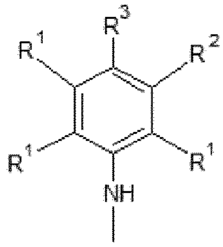

dans lequel $R^1$ et $R^2$ indépendamment l'un de l'autre sont H ou F, et $R^3$ est un groupe Z ; ou
dans lequel chaque $R^1$ est indépendamment H ou F, $R^2$ est un groupe Z, et $R^3$ est $-NO_2$;
dans lequel le groupe Z est choisi parmi $-C(O)NH-R_a$, $-S(O)_2NH-R_a$ et $-P(O)_2NH-R_a$ dans lequel $R_a$ est une chaîne alkyle en $C_1$-$C_4$ saturée linéaire ou ramifiée éventuellement substituée par un ou plusieurs groupes fonctionnels polaires choisis parmi -OH, -COOH, $-SO_3$, $-PO_4$, $-NH_2$, $-NH_4$+ ou avec un groupe perfluoroalkyle $-(CF_2)_m-CF_3$ dans lequel m est 1 à 70 ; ou dans lequel $R_a$ est - $(CHRCH_2O)_n$-X dans lequel n est 1 à 70, R est H ou $-CH_3$ et X est choisi parmi H, une chaîne alkyle en $C_1$-$C_4$ saturée ramifiée ou linéaire ou un groupe $-(CH_2)_p-O-(CH_2)_q$-W dans lequel W est H,$-CH_3$ ou $-NH_2$ et dans lequel p et q sont indépendamment 1 à 30 ; ou dans lequel $R_a$ est une chaîne alkyle en $C_1$-$C_{70}$ saturée linéaire ou ramifiée ou un groupe aromatique.

2. Dispositif médical selon la revendication 1, dans lequel la chaîne alkyle saturée linéaire ou ramifiée à l'intérieur de $R_a$ est choisie dans le groupe comprenant le polyéthylène, le polypropylène et les polyoléfines.

3. Dispositif médical selon l'une quelconque des revendications 1 ou 2, dans lequel le groupe aromatique de $R_a$ est choisi parmi le groupe comprenant le xylène, le polystyrène et l'acrylonitrile butadiène styrène et/ou dans lequel le groupe perfluoroalkyle à l'intérieur de $R_a$ est de formule-$(CF_2)_nCF_3$ dans lequel n= est de 1 à 70, et/ou dans lequel le groupe perfluoroalkyle est le polytetrafluoroéthylène.

4. Dispositif médical selon l'une quelconque des revendications précédentes, dans lequel à l'intérieur du groupe Z, $R_a$ est choisi parmi $-(CH_2CH_2)OH$, $-(CH_2CH_2CH_2)OH$,$-C(CH_2OH)_3$, $-(CH(CH_3)CH_2O)_9-CH_2CH_2OCH_3$, $-(CH_2)(CF_2)_7CF_3$.

5. Dispositif médical selon l'une quelconque des revendications précédentes, dans lequel le matériau polymère est choisi dans le groupe composé de copolymères à base de polyamide de formule générale

$$H-(O-PF-OOC-PA-COO-PF-OOC-PA-CO)_n-OH$$

dans lequel PA est un segment de polyamide et PF est un segment de diol comprenant des polyesters diol dimères à terminaison OH et n est entre 5 et 20 et un élastomère obtenu par polymérisation d'un composé de bloc formant un polyamide choisi dans le groupe comprenant l'acide aminocarboxylique de formule (1) ci-dessous et un lactame de formule (2) ci-dessous

$H_2N-R^1-COOH$ (1)

 (2)

avec un polyétherdiaminique tribloc de formule (3) ci-dessous :

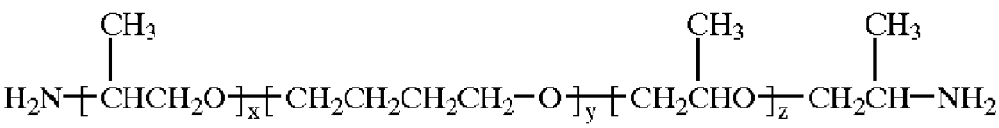 (3)

et un acide carboxylique de formule (4) ci-dessous :

$$HOOC-(R^3)_m-COOH \quad (4)$$

dans lequel $R^1$, $R^2$ et $R^3$ sont chacun des groupes de liaison comprenant une chaîne hydrocarbonée dans ceux-ci, qui peut être interrompue par un ou plusieurs groupes amide et dans lequel R1 et R2 comprennent indépendamment un groupe alcylène ayant de 2 à 20 atomes de carbone et des liaisons amide et R3 comprend un groupe alcylène ayant de 1 à 20 atomes de carbone et dans lequel x peut changer de 1 à 20, y peut changer de 4 à 50, Z peut changer de 1 à 20 et dans lequel m est 0 ou 1.

**6.** Dispositif médical selon l'une quelconque des revendications précédentes, dans lequel le tube de cathéter est un tube de fil-guide ou un tube de gonflage et dans lequel ladite surface comprend des groupes hydrophiles, et/ou dans lequel le tube de cathéter est un tube de fil-guide ou un tube d'aspiration et dans lequel ladite surface comprend des groupes hydrophobes, et/ou dans lequel la surface du tube de cathéter est la surface interne, et/ou dans lequel la surface du tube de cathéter est la surface externe et/ou dans lequel la surface du tube cathéter est la surface interne et externe.

**7.** Procédé permettant de modifier la surface du dispositif médical selon l'une quelconque des revendications précédentes, ledit procédé comprenant les étapes suivantes :

a) préparation d'une solution d'un composé de formule (I)

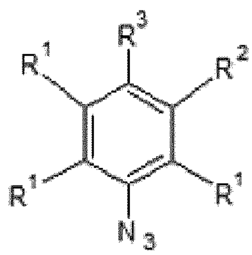

dans lequel $R^1$ et $R^2$ indépendamment l'un de l'autre sont H ou F, et $R^3$ est un groupe Z ; ou
dans lequel chaque $R^1$ est indépendamment H ou F, $R^2$ est un groupe Z, et $R_3$ est $-NO_2$;
dans lequel le groupe Z est choisi parmi $-C(O)NH-R_a$,$-S(O)_2NH-R_a$ et $-P(O)_2NH-R_a$ dans lequel $R_a$ est une chaîne alkyle en $C_1$-$C_4$ saturée linéaire ou ramifiée éventuellement substituée avec un ou plusieurs groupes fonctionnels polaires choisis parmi -OH, -COOH, $-SO_3$, $-PO_4$, $-NH_2$, $-NH_4$+ ou avec un groupe perfluoroalkyle $-(CF_2)_m-CF_3$ dans lequel m est 1 à 70 ;
ou dans lequel $R_a$ est $-(CHRCH_2O)_n-X$ dans lequel n est 1 à 70, R est H ou $-CH_3$ et X est choisi parmi H, une chaîne alkyle en $C_1$-$C_4$ saturée linéaire ou ramifiée ou un groupe $-(CH_2)_p-O-(CH_2)_q-W$ dans lequel W est H, $-CH_3$ ou $-NH_2$ et dans lequel p et q sont indépendamment 1 à 30 ; ou dans lequel $R_a$ est une chaîne alkyle en $C_1$-$C_{70}$ saturée linéaire ou ramifiée ou un groupe aromatique ; dans un solvant approprié ;

b) la mise en contact de la surface du dispositif médical avec la solution préparée à l'étape a) ;
c) l'irradiation de la surface obtenue à l'étape b) avec un rayonnement pouvant photoactiver ladite surface.

**8.** Procédé selon la revendication 7, dans lequel le composé de formule (I) est choisi dans le groupe comprenant :

le N-(tris(hydroxyméthyl)-4-azidobenzènesulphonylamide, le N(-2-hydroxyéthyl)-4-azidobenzamide, le N-(2-hydroxyéthyl)-4-azidobenzène sulphonylamide et

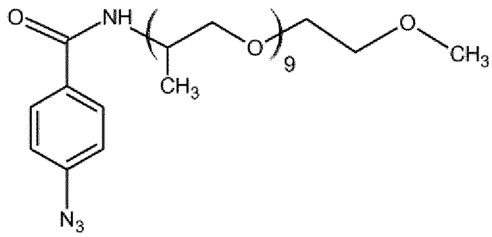

' ,

le 4-azido-2,3,5,6-tetrafluoro-N-(3-hydroxypropyl)benzamide, le 4-azido-2,3,5,6-tetrafluoro-N-(4,4,5,5,6,6,7,7, 8,8,9,9,10,10,11,11,11-heptadecafluoroundécyl)benzamide, la 2-nitro-5-azidobenzoylglycine, le N-(4,4,5,5,6, 6,7,7,8,8,9,9,10,10,11,11,11-heptadeca fluoroundécyl)5-azido-2-nitrobenzoate, le N-(tris(hydroxyméthyl)-5-azido-2-nitrobenzoate, le N-(dodecaéthylèneglycolmonométhyléther)-5-azido-2-nitrobenzoate.

**9.** Procédé selon l'une quelconque des revendications 7 ou 8, dans lequel le solvant de l'étape a) est choisi parmi le méthanol, l'éthanol, l'acétonitrile et le chloroforme, et/ou dans lequel ledit rayonnement possède une longueur d' onde qui est capable de passer à travers le matériau polymère du dispositif médical, et/ou dans lequel ledit rayonnement a une longueur d'onde comprise entre 200 à 600 nm, de préférence entre 250 à 350 nm et mieux encore entre 230 à 300 nm, et/ou dans lequel l'étape d'irradiation c) est réalisée dans un environnement sombre à température ambiante et/ou dans lequel l'étape d'irradiation c) est réalisée pendant 0,5 à 1 heure.

**10.** Procédé selon l'une quelconque des revendications 7 à 9, dans lequel l'étape b) est réalisée en passant la solution comprenant le composé de formule (I) à travers la surface du dispositif médical, et/ou comprenant, avant l'étape b), une étape dans laquelle la surface qui doit être traitée est lavée et séchée, et/ou dans lequel après l'étape b), la surface est lavée afin d'éliminer les composés non liés de formule (I) et/ou dans lequel ladite surface du dispositif médical est la surface interne, la surface externe ou les deux.

**11.** Composé de formule (I) qui est
le N-(tris(hydroxyméthyl)-4-azidobenzènesulphonylamide, le N-(2-hydroxyéthyl)-4-azidobenzènesulphonylamide,

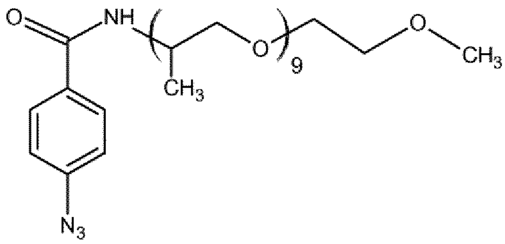

,

le N-(4,4,5,5,6,6,7,7,8,8,9,9,10,10,11,11,11-heptadécafluoroundécyl)-5-azido-2-nitrobenzoate, le N-(tris(hydroxyméthyl)-5-azido-2-nitrobenzoate ou le N-(dodecaéthylèneglycolmonométhyléther)-5-azido-2-nitrobenzoate.

**12.** Utilisation d'un composé selon la revendication 11, pour modifier les propriétés d'une surface polymère.

**13.** Utilisation d'un composé selon la revendication 12, dans laquelle ladite surface polymère est la surface d'un dispositif médical, et/ou dans laquelle ladite surface est la surface interne et/ou la surface externe d'un dispositif médical.

**14.** Utilisation d'un composé selon l'une quelconque des revendications 12 à 13, dans laquelle ladite surface est la surface interne d'un ballon d'un cathéter ; ou dans laquelle ladite surface est la surface interne et/ou la surface externe d'un tube de cathéter dans laquelle généralement ledit tube de cathéter est choisi parmi un tube de fil-guide, un tube de gonflement et un tube d'aspiration.

FIG. 1

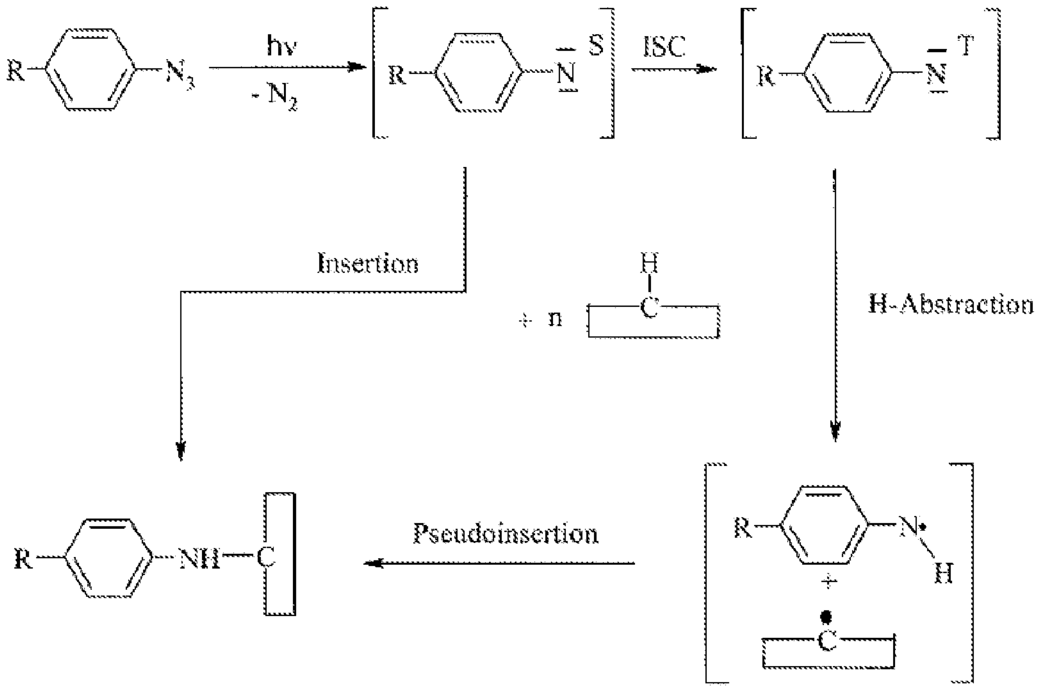

FIG. 2

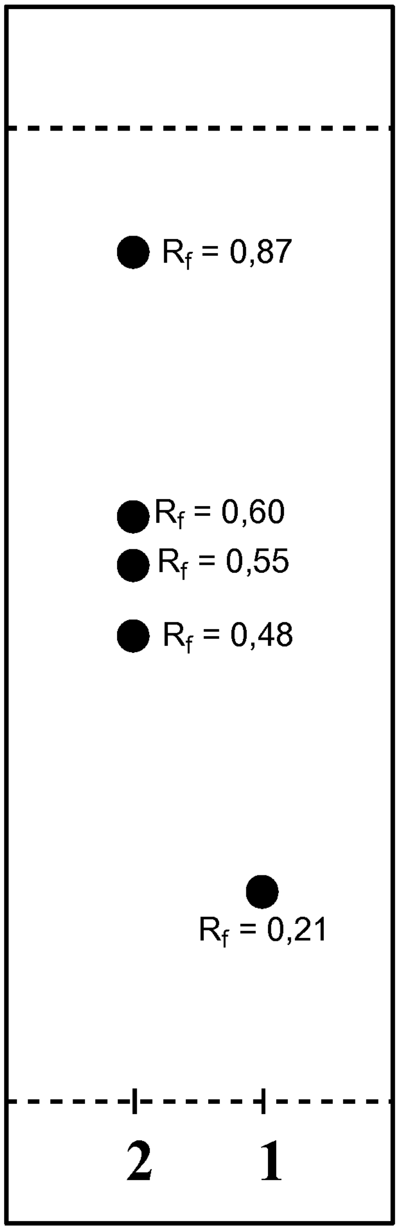

FIG. 3

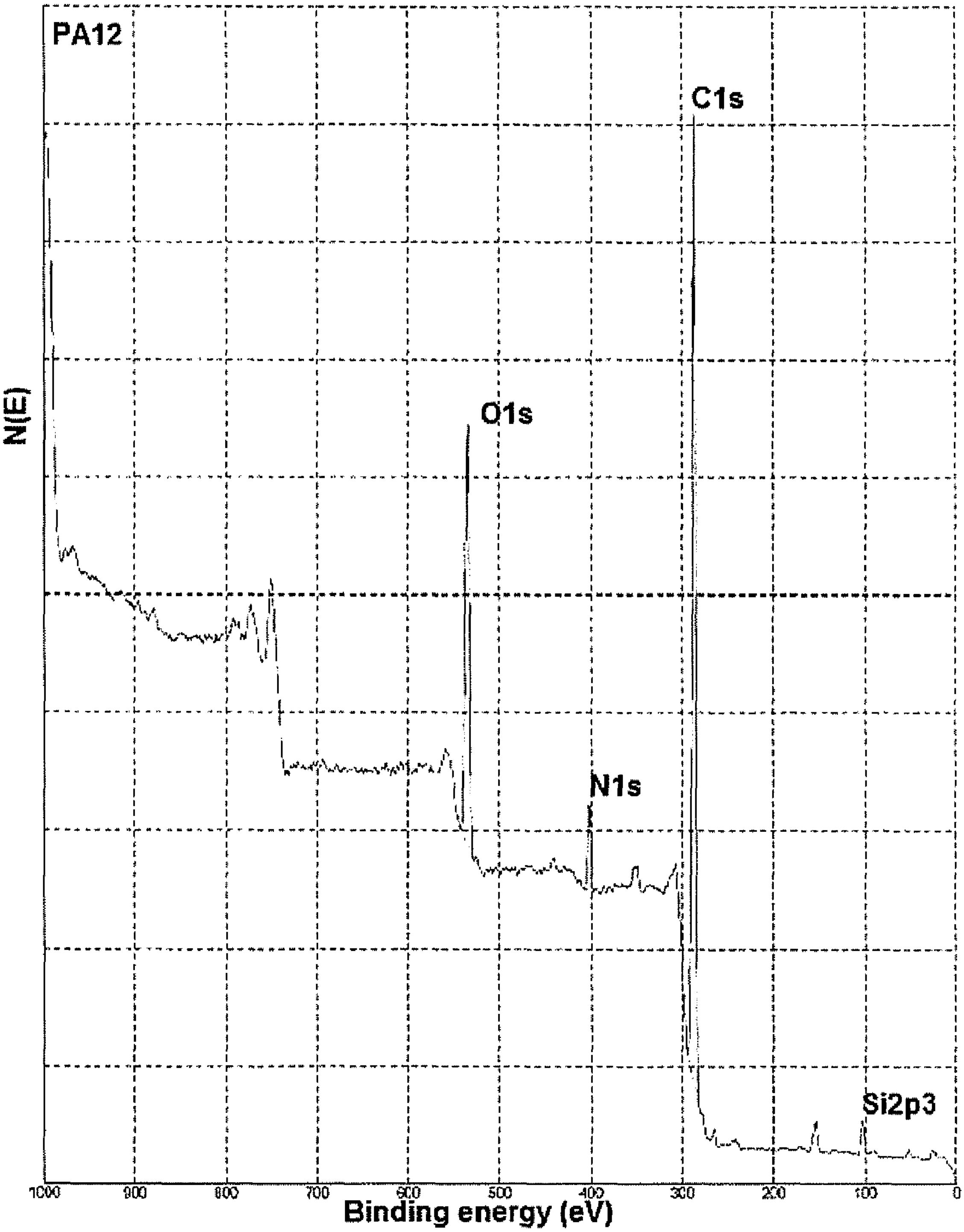
PA12
C1s
O1s
N1s
Si2p3
N(E)
1000
900
800
700
600
500
400
300
200
100
0
Binding energy (eV)

FIG. 4

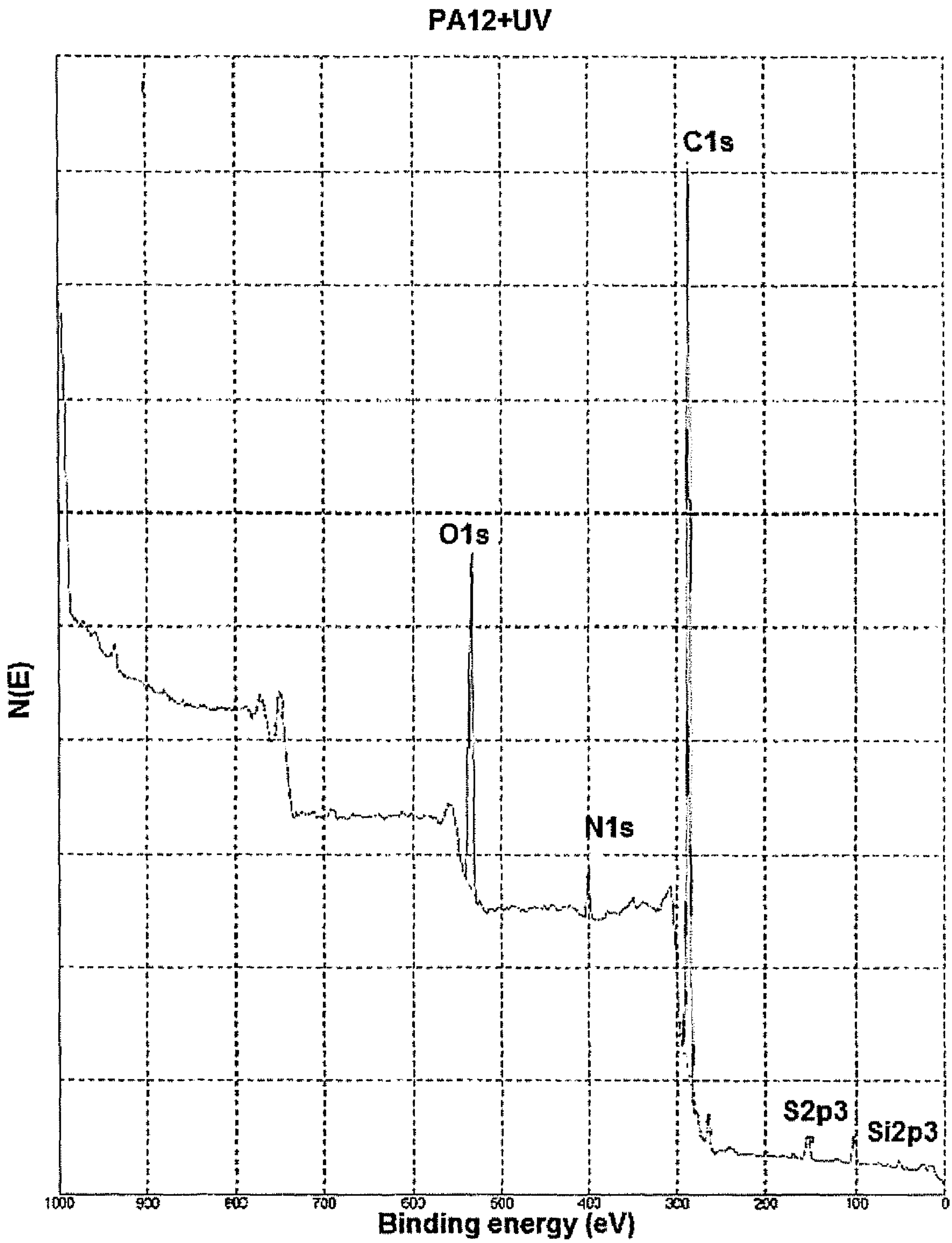
PA12+UV
C1s
O1s
N1s
S2p3
Si2p3
N(E)
1000
900
800
700
600
500
400
300
200
100
0
Binding energy (eV)

FIG. 5

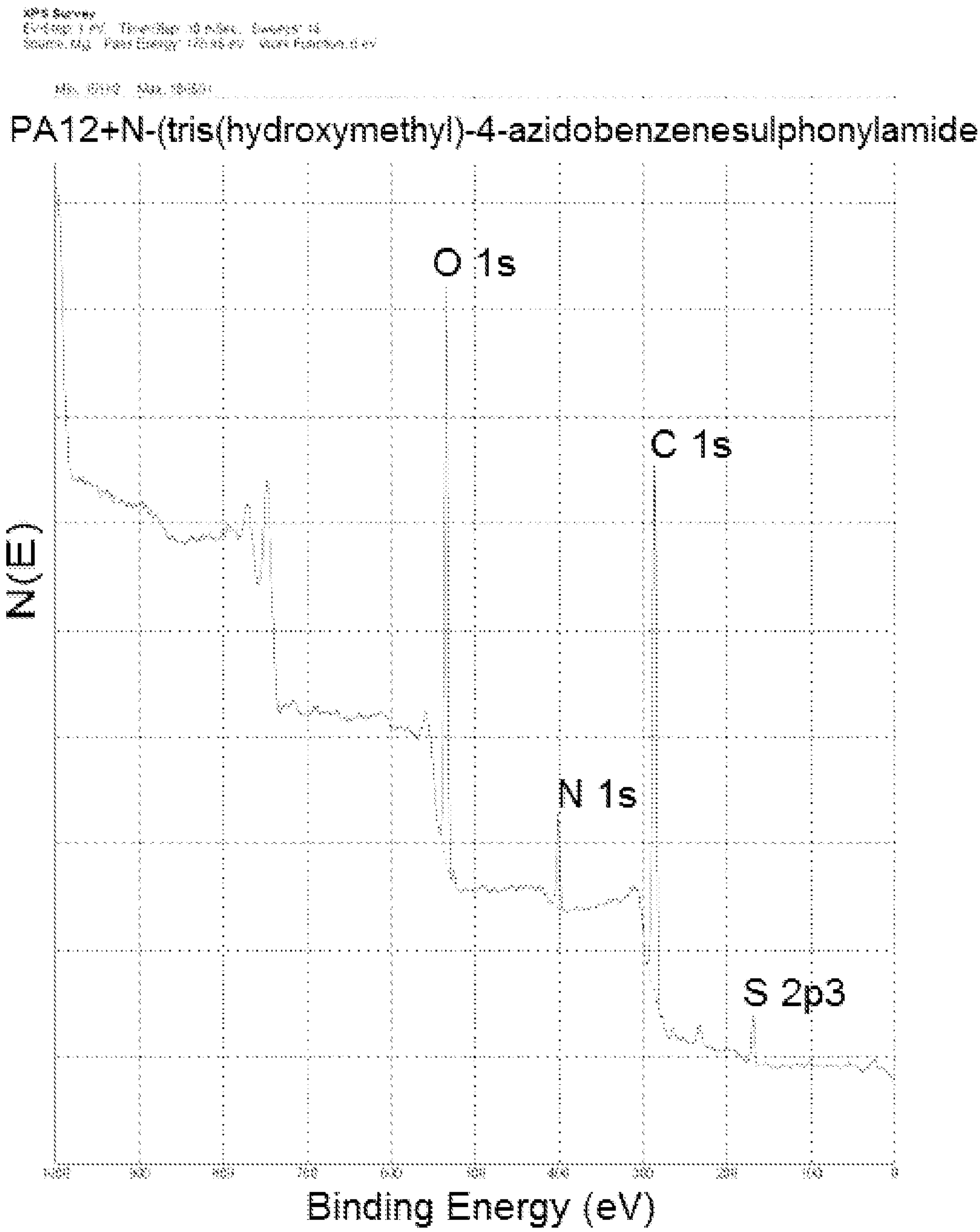
PA12+N-(tris(hydroxymethyl)-4-azidobenzenesulphonylamide
O 1s
C 1s
N 1s
S 2p3
N(E)
Binding Energy (eV)

FIG. 6

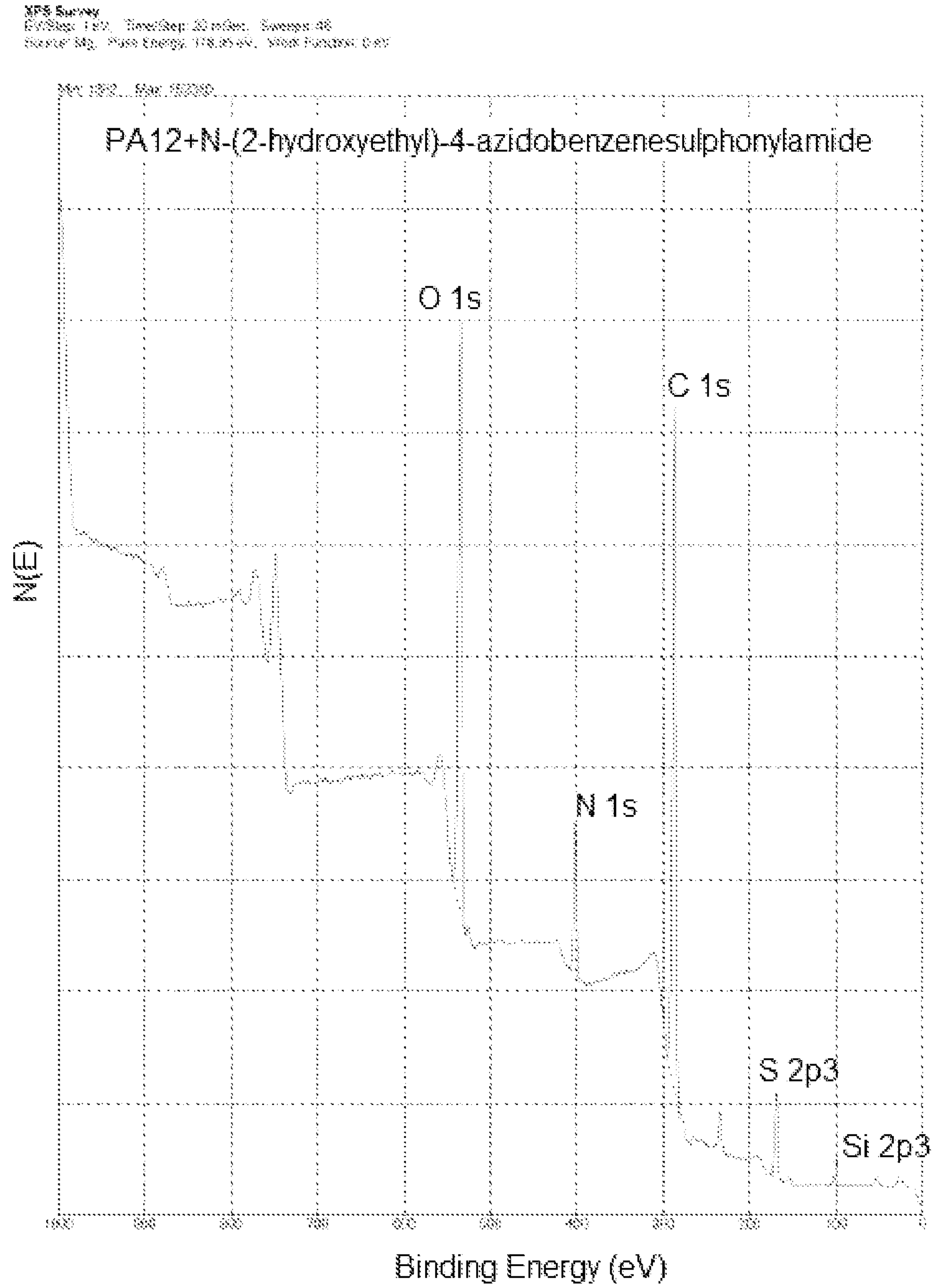
XPS Survey
PA12+N-(2-hydroxyethyl)-4-azidobenzenesulphonylamide
O 1s
C 1s
N 1s
S 2p3
Si 2p3
N(E)
Binding Energy (eV)

FIG. 7

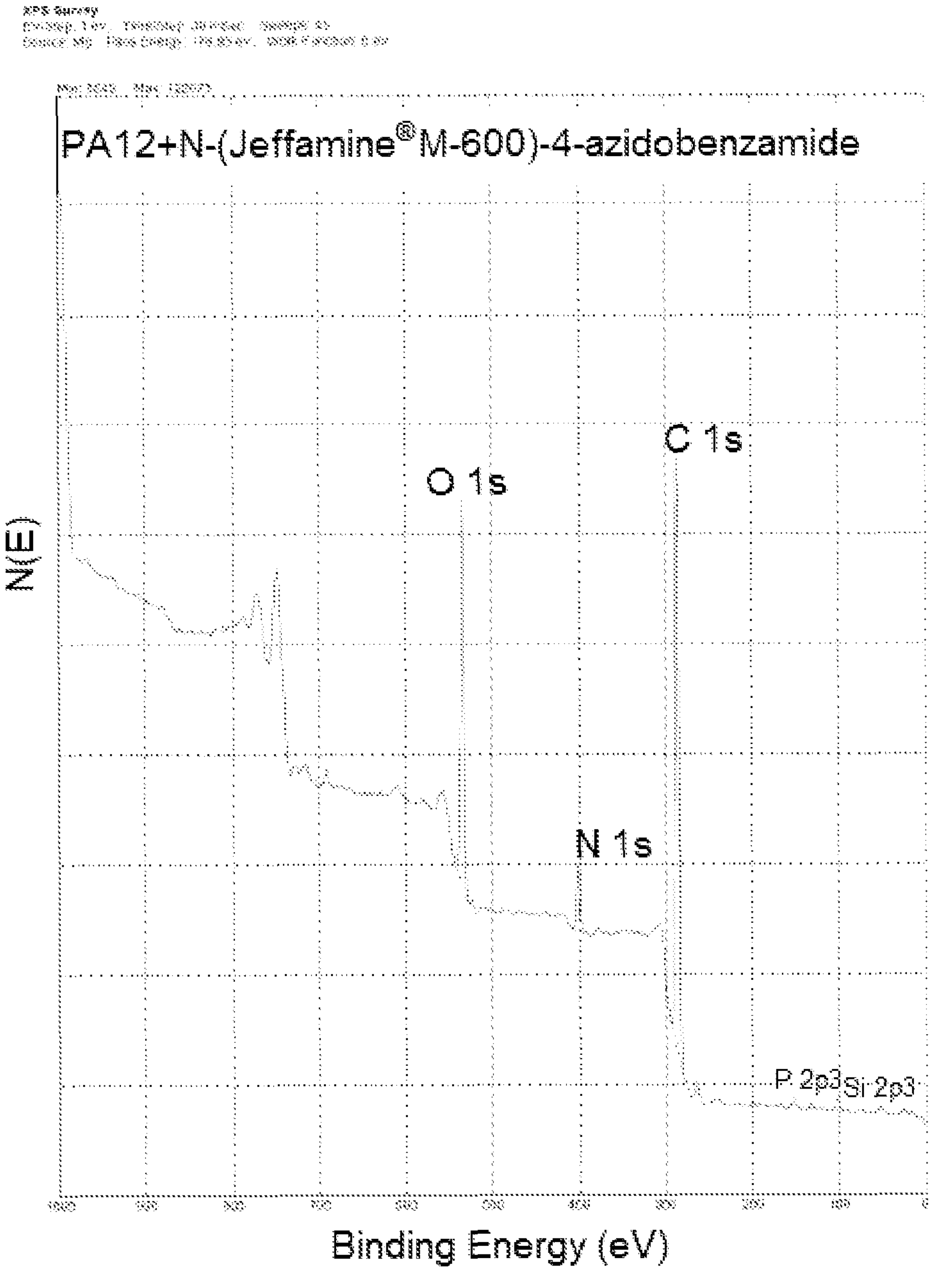
PA12+N-(Jeffamine®M-600)-4-azidobenzamide
C 1s
O 1s
N 1s
P 2p3
Si 2p3
N(E)
Binding Energy (eV)

FIG. 8

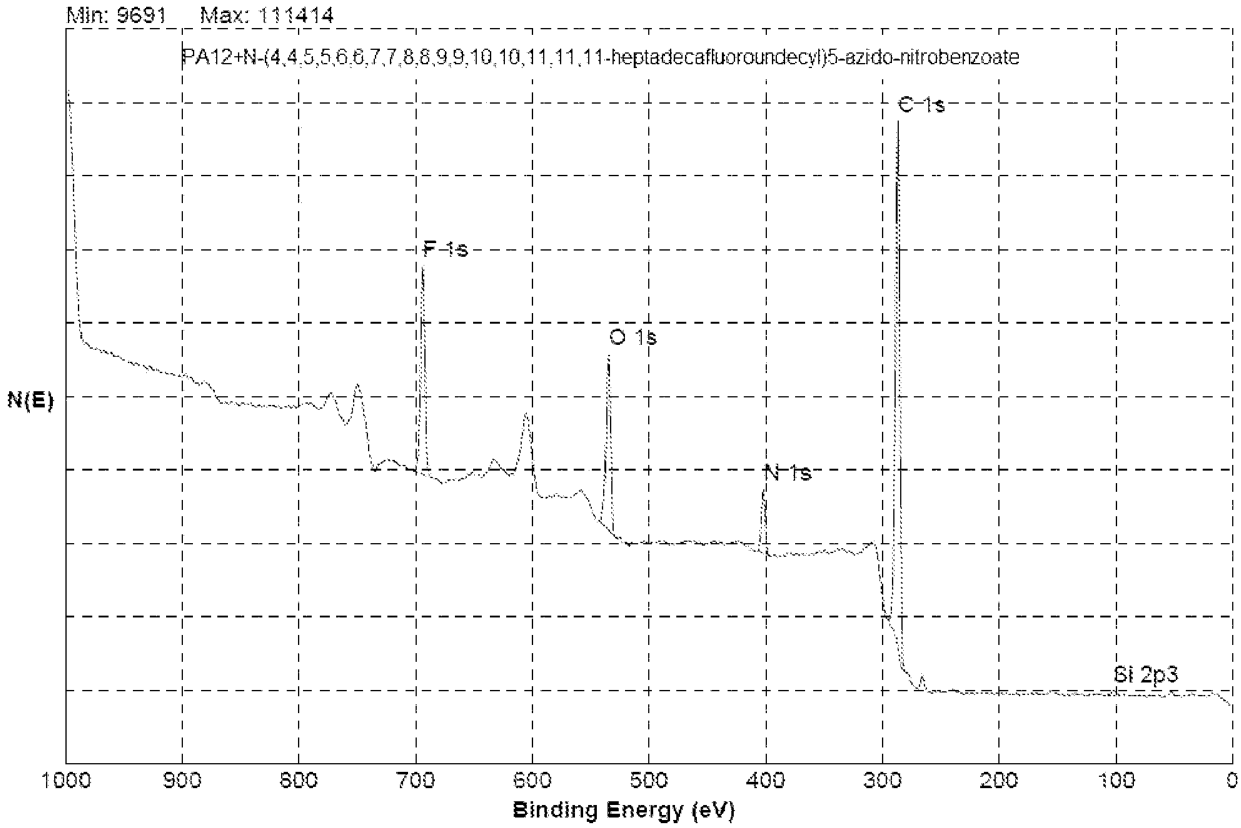
Min: 9691 Max: 111414
PA12+N-(4,4,5,5,6,6,7,7,8,8,9,9,10,10,11,11,11-heptadecafluoroundecyl)5-azido-nitrobenzoate
C 1s
F 1s
O 1s
N 1s
Si 2p3
N(E)
1000
900
800
700
600
500
400
300
200
100
0
Binding Energy (eV)

FIG. 9

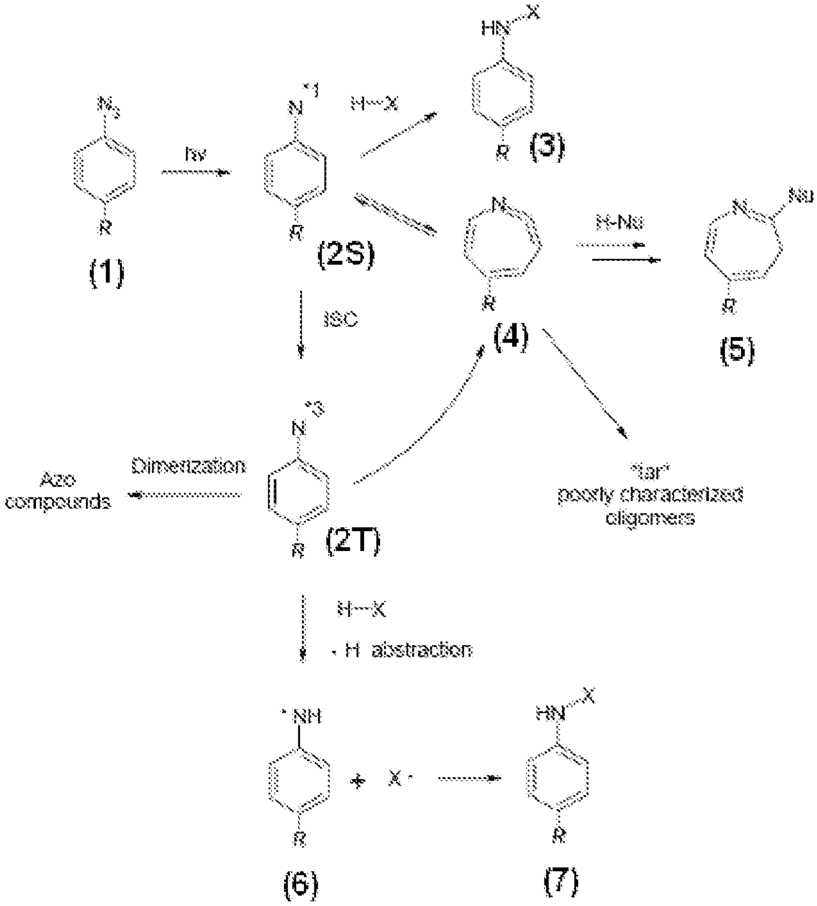

(1) (2S) (3) (4) (5) (2T) (6) (7)

FIG. 10

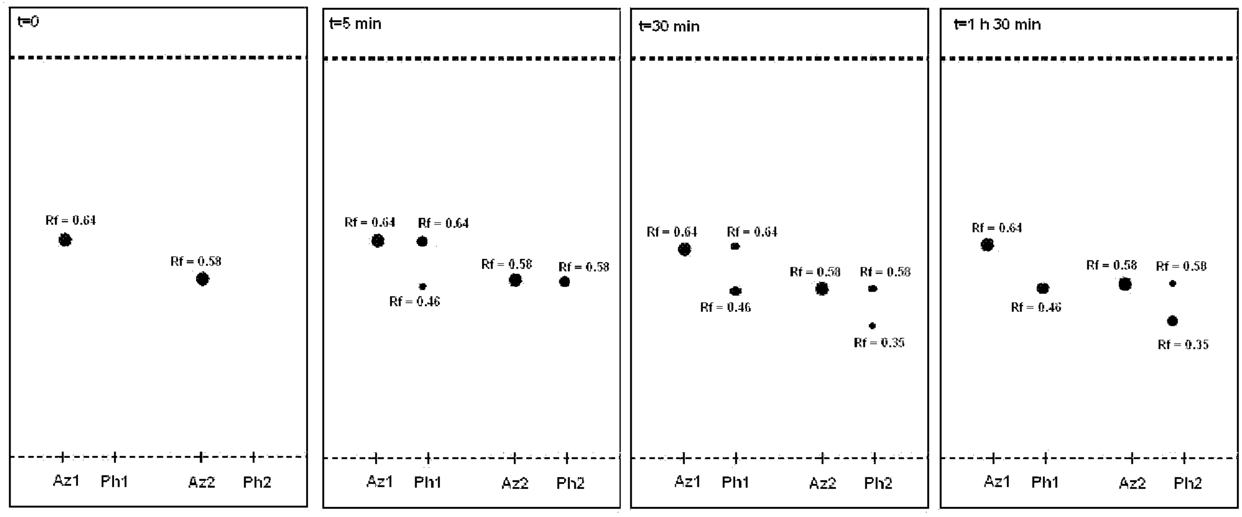
t=0
Rf = 0.64
Rf = 0.58
Az1
Ph1
Az2
Ph2
t=5 min
Rf = 0.64
Rf = 0.64
Rf = 0.46
Rf = 0.58
Rf = 0.58
Az1
Ph1
Az2
Ph2
t=30 min
Rf = 0.64
Rf = 0.64
Rf = 0.46
Rf = 0.58
Rf = 0.58
Rf = 0.35
Az1
Ph1
Az2
Ph2
t=1 h 30 min
Rf = 0.64
Rf = 0.46
Rf = 0.58
Rf = 0.58
Rf = 0.35
Az1
Ph1
Az2
Ph2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007056338 A2
- WO 2004056909 A1
- EP 0633031 A1
- US 5714360 A
- US 6551267 B
- US 6120904 A
- US 5415619 A
- US 5061738 A
- US 6506333 B
- WO 2005037337 A
- WO 2007132485 A


### Non-patent literature cited in the description


- **SMOKAL et al.** *Digest Journal of Nanomaterials and Biostructures,* 2008, vol. 3 (1), 41-47
- *Polymer,* 2006, vol. 47 (14), 4916-4924
- **FENG SHI et al.** *Langmuir,* 2007, vol. 23 (3), 1253-1257
- **HEIDEN et al.** *Journal of Biomedical Materials Research,* 1998, vol. 40 (2), 195-203
- **G.A. WIESE ; J. W. JONES.** Preparation of Tris (hydroxymethyl) sulfanilamidomethane. *Journal of the American Pharmaceutical Association,S.E.,* 1948, 380-383
- **H.BRINTZINGER ; H. KODDEBUSCH.** Amid- und Ester-amid-Bildung zwischen carbonsäurechloriden und Mono-, Di- und Triäthanolamin. *Chemische Berichte,* 1949, vol. 82, 201
- **M.L. CROSSLEY ; E.H. NORTHEY ; M.E. HULTQUIST.** Sulfanilamide Derivatives. VI. Substituted N-Aliphatic Sulfaniamides. *J. Am. Chem. Soc.,* 1940, vol. 62 (3), 532-534
- **S. J. PASTINE ; D. OKAWA ; B. KESSLER ; M. ROLANDI ; M. LLORENTE ; A. ZETTL ; J. M. J. FRECHET.** Facile UV Patterning of Robust Carbon Nanotube Forests Using Perfluoroarylazides. *J. AM. CHEM. SOC.,* 2008, vol. 130, 4238-4239
- **R. E. GALARDY ; L. C. CRAIG ; J. D. JAMIESON ; M.P. PRINTZ.** Photoaffinity Labeling of Peptide Hormone Binding Sites. *The Journal of Biological Chemestry,* 1974, vol. 249 (11), 2510-2618
- **CLAUDIO SALVAGNINI.** *Thrombin inhibitors grafting on polyester membranes for the preparation of blood-compatible materials,* 2005